# DEMANDE DE BREVET EUROPEEN

(11) **EP 3 395 272 A1**
(43) Date de publication de la demande: **31.10.2018**
(21) Numéro de dépôt: 18167852.5
(22) Date de dépôt: 17.04.2018
(51) Int. Cl.: A61B 17/72

(54) **IMPLANT D'ARTHRODÈSE ET KIT CHIRURGICAL COMPRENANT UN TEL IMPLANT**

(30) Priorité: 25.04.2017 FR 1753605
(71) Demandeur: In2Bones, 69130 Ecully (FR)
(72) Inventeur: PAPALOÏZOS, Michaël, 1204 GENEVE (CH)
(74) Mandataire: Weber, Jean-François

(57) **Abrégé**

- Implant d'arthrodèse et kit chirurgical comprenant un tel implant.
- L'invention concerne un implant (1) d'arthrodèse pour la fusion d'un premier et d'un deuxième os (2, 3), ledit implant (1) comprenant un composant primaire (4) comportant un corps d'ancrage primaire (5) et un connecteur mâle (6), et un composant secondaire (7) comportant un corps d'ancrage secondaire (8) et un connecteur femelle (9), ce dernier comprenant :
- un corps (10) s'étendant entre une extrémité arrière (11) et une face avant (12), et présentant une paroi latérale (14) reliant ces dernières ;
- un logement (15) ménagé dans le corps (10) et configuré pour recevoir le connecteur mâle (6),
le connecteur femelle (9) comprenant une ouverture (18) ménagée dans le corps (10) et s'étendant de manière continue sur lesdites face avant (12) et paroi latérale (14), et étant dimensionnée et configurée pour autoriser une introduction latérale du connecteur mâle (6) dans le logement (15),
les composant primaire (4) et secondaire (6) étant chacun monobloc, et les corps d'ancrage primaire (5) et secondaire (8) étant respectivement conçus pour assurer une insertion et un ancrage du composant primaire (4) dans le premier os (2), et du composant secondaire (7) dans le deuxième os (3), par impaction ou coincement en force.
- Implants chirurgicaux d'ostéosynthèse.

## Description

L'invention se rapporte au domaine général des implants chirurgicaux d'ostéosynthèse, et en particulier aux implants phalangiens destinés à être mis en place dans le corps d'un patient, au niveau de la main ou du pied de ce dernier, afin de permettre la fusion de deux phalanges. L'invention se rapporte également aux domaines des kits chirurgicaux comprenant de tels implants.

L'invention concerne plus précisément un implant d'arthrodèse pour favoriser la fusion osseuse d'un premier os avec un deuxième os, ledit implant comprenant un composant primaire comportant un corps d'ancrage primaire et un connecteur mâle, et un composant secondaire comportant un corps d'ancrage secondaire et un connecteur femelle, ledit connecteur femelle comprenant :
- un corps principal qui s'étend longitudinalement entre une extrémité arrière reliée audit corps d'ancrage secondaire et une face avant opposée, et qui présente une paroi latérale reliant ladite extrémité arrière à ladite face avant ;
- un logement ménagé au sein dudit corps principal et configuré pour recevoir ledit connecteur mâle afin de relier ensemble lesdits composants primaire et secondaire.

L'invention concerne également un kit chirurgical comprenant un implant d'arthrodèse pour favoriser la fusion osseuse d'un premier os avec un deuxième os.

Afin de traiter certaines pathologies osseuses, en particulier des phalanges, telles que des déformations inter-phalangiennes, des orteils en griffe ou en marteau, ou encore de l'arthrose, on est parfois amené à effectuer une arthrodèse, c'est-à-dire à provoquer une fusion interosseuse pour fixer un os par rapport à l'autre. Dans le cas des phalanges par exemple, il est connu de mettre en place un implant intra-médullaire au cours d'une intervention chirurgicale, qui vise à solidariser deux phalanges l'une par rapport à l'autre, afin d'entraîner leur fusion osseuse. De tels implants d'arthrodèse connus sont le plus souvent réalisés de manière à former une pièce d'un seul tenant, ce qui permet d'améliorer leur longévité dans le corps du patient, de manière par exemple à ne pas nécessiter d'être retirés dudit corps. Alternativement, on connaît également des implants d'arthrodèse comportant, au contraire, deux composants distincts complémentaires, à savoir un composant primaire destiné à être ancré dans le canal médullaire de l'une des deux phalanges, et un composant secondaire qui peut être ancré dans le canal médullaire de la deuxième phalange. Ces composants primaire et secondaire comportent des connecteurs complémentaires conçus pour relier et verrouiller ensemble lesdits composants primaire et secondaire.

Si de tels implants connus donnent généralement satisfaction, ils n'en présentent pas moins certains inconvénients et s'avèrent perfectibles. En particulier, la pose de ces implants connus peut parfois s'avérer difficile pour le chirurgien et traumatique pour le patient. En effet, leur mise en place dans le corps du patient nécessite que le chirurgien procède au préalable à une distraction articulaire (c'est-à-dire à un écartement) relativement importante des phalanges, afin de pouvoir assurer la mise en correspondance des connecteurs mâle et femelle et le couplage des composants primaire et secondaire. Or une telle distraction des phalanges peut s'avérer délétère pour les tendons et tissus mous environnants, conduisant potentiellement à des douleurs importantes pour le patient et à l'allongement de la période de rémission de ce dernier.

Les objets assignés à la présente invention visent en conséquence à remédier aux différents inconvénients énumérés précédemment et à proposer un nouvel implant d'arthrodèse dont la mise en place est à la fois rapide, facile et particulièrement peu traumatisante pour le patient, en particulier pour les tendons et tissus mous environnants l'articulation à traiter.

Un autre objet de l'invention vise à proposer un nouvel implant particulièrement robuste et résistant, et dont la tenue dans le corps du patient est particulièrement fiable.

Un autre objet de l'invention vise à proposer un nouvel implant polyvalent pour s'adapter à la morphologie de chaque patient tout en permettant une arthrodèse relativement rapide et indolore.

Un autre objet de l'invention vise à proposer un nouvel implant dont la fabrication est relativement aisée et peu onéreuse.

Un autre objet de l'invention vise à proposer un nouvel implant permettant de réduire le coût de l'intervention chirurgicale de pose de l'implant, tout en réduisant le risque pour la santé du patient lié à la pose de l'implant et à la thérapie.

Un autre objet de l'invention vise à proposer un nouvel implant permettant de traiter une pathologie osseuse du patient de façon particulièrement efficace, et rapide.

Un autre objet de l'invention vise à proposer un nouveau kit chirurgical comprenant un tel implant amélioré ainsi que des instruments chirurgicaux permettant une pose aisée, rapide et précise de ce dernier dans le corps d'un patient.

Les objets assignés à l'invention sont atteints à l'aide d'un implant d'arthrodèse pour favoriser la fusion osseuse d'un premier os avec un deuxième os, ledit implant comprenant un composant primaire comportant un corps d'ancrage primaire et un connecteur mâle, et un composant secondaire comportant un corps d'ancrage secondaire et un connecteur femelle, ledit connecteur femelle comprenant :
- un corps principal qui s'étend longitudinalement entre une extrémité arrière reliée audit corps d'ancrage secondaire et une face avant opposée, et qui présente une paroi latérale reliant ladite extrémité arrière à ladite face avant ;
- un logement ménagé au sein dudit corps principal et configuré pour recevoir ledit connecteur mâle afin de relier ensemble lesdits composants primaire et secondaire,
ledit connecteur femelle comprenant en outre une ouverture qui est ménagée dans ledit corps principal, en communication avec ledit logement, et qui s'étend de manière continue à la fois sur ladite face avant et ladite paroi latérale, et qui est dimensionnée et configurée pour autoriser une introduction latérale du connecteur mâle dans ledit logement par ladite ouverture,
lesdits composant primaire et secondaire formant chacun une pièce monobloc d'un seul tenant, et lesdits corps d'ancrage primaire et secondaire étant respectivement conçus pour assurer une insertion et un ancrage du composant primaire dans le premier os, et du composant secondaire dans le deuxième os, par impaction ou par coincement en force.

Les objets de l'invention sont également atteints à l'aide d'un kit chirurgical comprenant un implant d'arthrodèse pour favoriser la fusion osseuse d'un premier os avec un deuxième os, ledit implant d'arthrodèse étant conforme à ce qui précède, et ledit kit comprenant également :
- un premier instrument de préhension pourvu d'un premier embout de solidarisation du composant primaire de l'implant avec le premier instrument de préhension, par l'intermédiaire du connecteur mâle du composant primaire ;
- un deuxième instrument de préhension pourvu d'un deuxième embout de solidarisation du composant secondaire de l'implant avec le deuxième instrument de préhension, par l'intermédiaire du connecteur femelle du composant secondaire.

D'autres particularités et avantages de l'invention apparaîtront et ressortiront plus en détails à la lecture de la description faite ci-après, en référence aux dessins annexés, donnés uniquement à titre d'exemples illustratifs et non limitatifs, dans lesquels :
- La figure 1 illustre, selon une vue de dessus, un premier mode de réalisation préférentiel de implant de l'invention ;
- Les figures 2 et 3 illustrent respectivement, selon des vues en perspective, deux variantes du composant primaire de l'implant de la figure 1. Les figures 4 à 6 illustrent, respectivement des vues de dessus, en perspective et en coupe longitudinale, le composant secondaire de la figure 1 ;
- Les figures 7 à 9 illustrent, selon des vues en perspective, des étapes successives de mise en oeuvre préférentielle de l'implant de la figure 1 ;
- Les figures 10 à 13 illustrent, selon des vues en perspective, des variantes du composant primaire de l'implant de l'invention, selon un deuxième mode de réalisation préférentiel de ce dernier. La figure 14 illustre, selon une vue en perspective, une variante préférentielle du composant secondaire de l'implant de l'invention selon ce deuxième mode de réalisation préférentiel ;
- Les figures 15 à 17 illustrent, selon des vues en perspective, des étapes successives de mise en oeuvre préférentielle de l'implant de l'invention, selon le deuxième mode de réalisation préférentiel de ce dernier ;
- La figure 18 illustre, selon une vue en perspective, le premier instrument de préhension du kit de l'invention, pourvu d'un premier embout de solidarisation du composant primaire d'un implant conforme à l'invention, La figure 19 illustre, selon une vue en perspective, le premier embout de ce premier instrument ;
- Les figures 20 et 21 illustrent, selon des vues en coupe longitudinale, des configurations déverrouillée (figure 20) et verrouillée (figure 21) du composant primaire d'un implant conforme à l'invention dans l'embout de la figure 19 ;
- La figure 22 illustre, selon une vue en perspective, le deuxième instrument de préhension du kit de l'invention, pourvu d'un deuxième embout de solidarisation du composant secondaire d'un implant conforme à l'invention. La figure 23 illustre, selon une vue en perspective, le deuxième embout de ce deuxième instrument ;
- Les figures 24 et 25 illustrent, selon des vues en coupe longitudinale, des configurations déverrouillée (figure 24) et verrouillée (figure 25) du composant secondaire d'un implant conforme à l'invention sur l'embout de la figure 23.

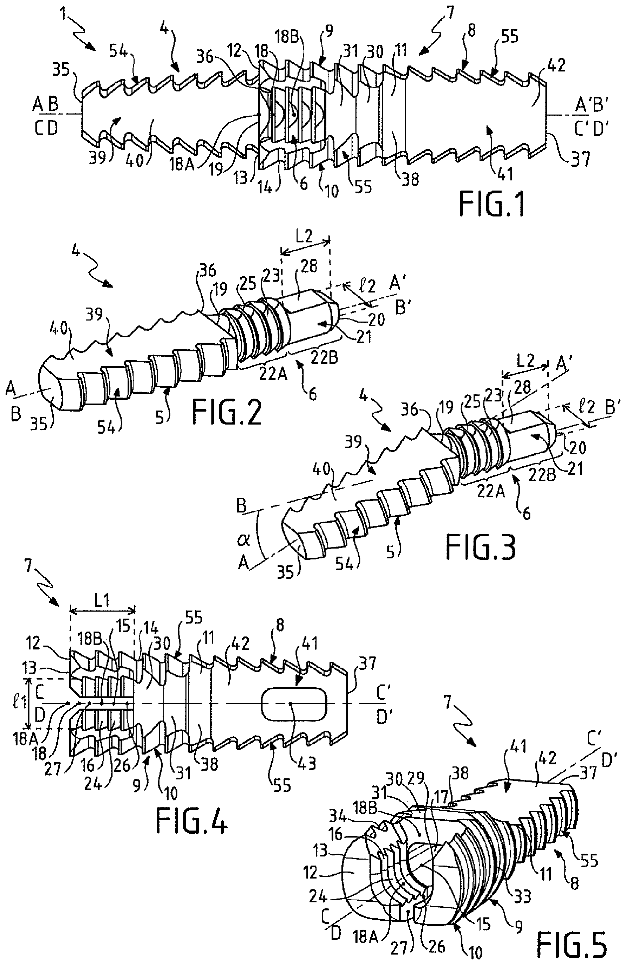

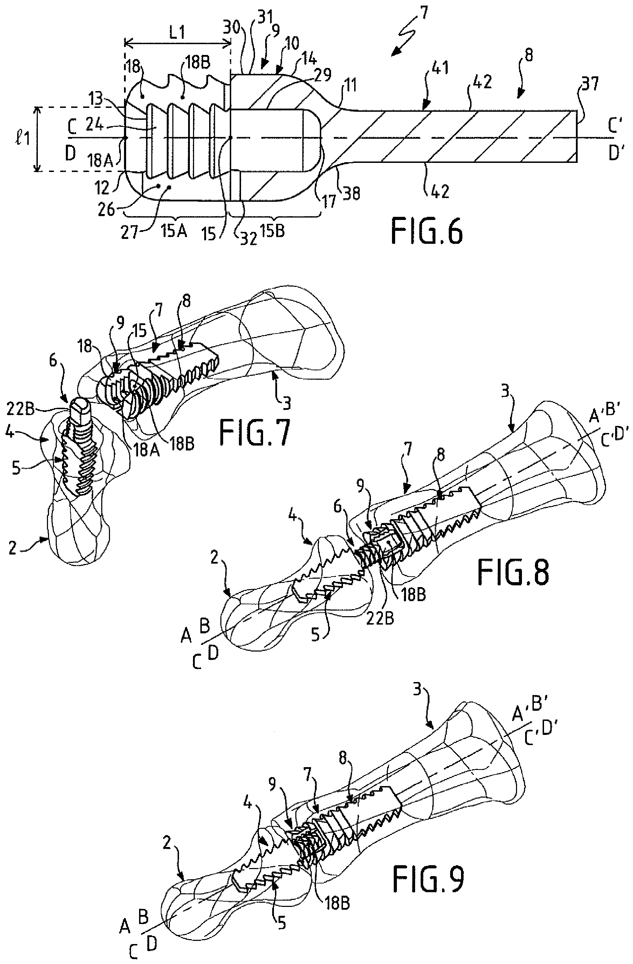

L'invention concerne en tant que tel un implant 1 d'arthrodèse (figure 1), c'est-à-dire un implant 1 chirurgical destiné à être mis en place dans le corps d'un patient, humain ou animal, au niveau de ses os, par exemple au cours d'une opération chirurgicale effectuée sous anesthésie locale ou générale. L'implant 1 de l'invention permet d'effectuer une arthrodèse d'un premier os 2 avec un deuxième os 3, c'est-à-dire qu'il permet de favoriser la fusion osseuse de ces premier 2 et deuxième os 3. La fusion osseuse est préférentiellement effectuée au niveau d'une articulation connectant le premier os 2 au deuxième os 3, de manière à supprimer cette dernière. Alternativement, l'implant de l'invention pourrait être mis en oeuvre pour favoriser la fusion osseuse entre deux corps osseux distincts provenant d'un même os (ou d'os différents) du patient. Une telle opération chirurgicale peut notamment permettre, dans le cas où le patient souffre par exemple d'une arthrose interarticulaire, de supprimer la douleur subie par le patient en fusionnant l'articulation atteinte. Alternativement, l'opération peut permettre de traiter par exemple une déformation inter-phalangienne, ou des orteils en griffe ou en marteau. Ainsi, l'implant 1 de l'invention peut avantageusement être inséré au niveau de l'articulation entre deux os de la colonne vertébrale, du genou, ou du coude. De préférence cependant, l'implant 1 de l'invention forme un implant intra-médullaire phalangien, c'est-à-dire un implant destiné à être mis en oeuvre au niveau de phalanges d'une main ou d'un pied d'un patient. Le premier os 2 forme ainsi une première phalange et le deuxième os 3 forme une deuxième phalange du même doigt ou orteil que la première phalange (figures 7 à 9 et 15 à 17). De manière encore plus précise, l'implant 1 a préférentiellement vocation à être positionné au niveau d'une articulation inter-phalangienne distale d'une main d'un patient, c'est-à-dire au niveau de l'articulation séparant les phalanges distale et intermédiaire, de préférence de l'index, du majeur, ou de l'annulaire.

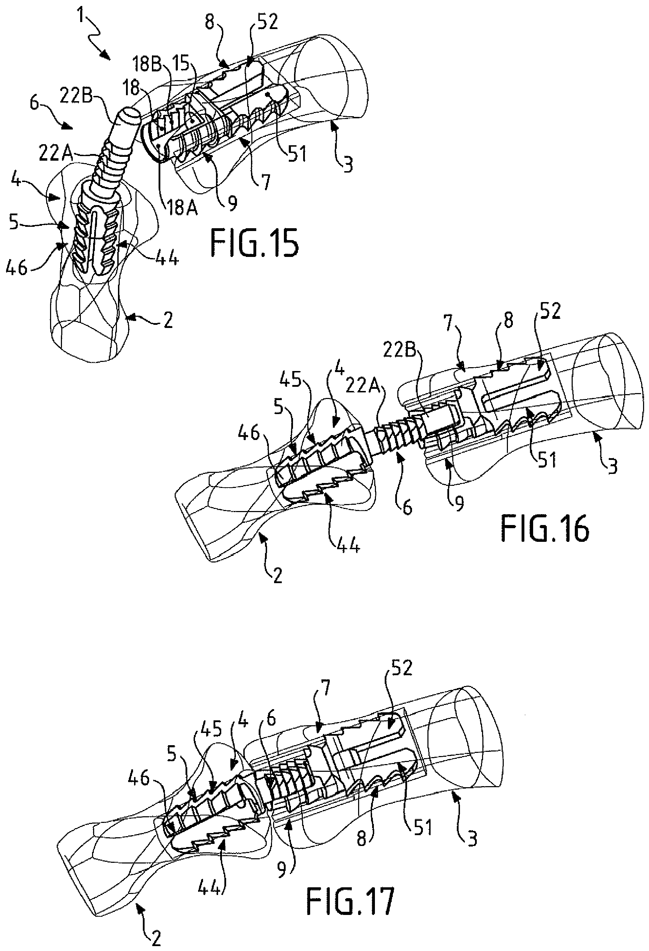

Tel qu'illustré aux figures, l'implant 1 de l'invention comprend deux composants distincts, conçus pour être reliés l'un à l'autre. En l'espèce, l'implant 1 comprend un composant primaire 4, qui comporte lui-même un corps d'ancrage primaire 5 avantageusement conçu pour assurer l'ancrage du composant primaire 4 dans le premier os 2, et un connecteur mâle 6. De préférence, ledit corps d'ancrage primaire 5 et ledit connecteur mâle 6 s'étendent respectivement selon des premier A-A' et deuxième B-B' axes d'extension longitudinale. L'implant 1 comprend également un composant secondaire 7, qui comporte quant à lui un corps d'ancrage secondaire 8 avantageusement conçu pour assurer l'ancrage du composant secondaire 7 dans le deuxième os 3, et un connecteur femelle 9. De préférence, ledit corps d'ancrage secondaire 8 et ledit connecteur femelle 9 s'étendent respectivement selon des troisième C-C' et quatrième D-D' axes d'extension longitudinale. Lesdits connecteurs mâle 6 et femelle 9 sont avantageusement conçus et configurés pour coopérer par complémentarité, de manière réversible ou non, pour relier ensemble lesdits composants primaire 4 et secondaire 7, de préférence en assurant une liaison mécanique directe, robuste et fiable, entre ces derniers. Autrement dit, si lesdits composants primaire 4 et secondaire 7 de l'implant 1 peuvent avantageusement être mis en oeuvre séparément, par exemple de manière séquentielle, au cours d'une même opération chirurgicale de pose de l'implant 1 dans le corps d'un patient, les composants primaire 4 et secondaire 7 sont cependant prévus pour être *in fine* reliés ensemble par l'intermédiaire des connecteurs mâle 6 et femelle 9 (figure 1). Ainsi, le premier os 2 et le deuxième os 3 sont reliés l'un à l'autre par l'implant 1, une fois lesdits composants primaire 4 et secondaire 7 reliés ensemble, et avantageusement solidarisés, par l'intermédiaire de leurs connecteurs mâle 6 et femelle 9 respectifs (figures 9 et 17).

De façon préférentielle, les composants primaire 4 et secondaire 6 forment chacun une pièce monobloc d'un seul tenant, les corps d'ancrage primaire 5 et secondaire 7 venant respectivement de matière avec les connecteurs mâle 6 et femelle 9. Une telle conception permet de conférer aux composants primaire 4 et secondaire 7 une bonne résistance structurelle tout en facilitant leur fabrication, par exemple à l'aide d'opération(s) de moulage et / ou d'usinage, sans nécessiter d'étape d'assemblage de pièces rapportées. Alternativement, lesdits composants primaire 4 et secondaire 7 pourront au contraire être le résultat de l'assemblage de plusieurs pièces, et ne pas être monoblocs. Les composants primaire 4 et secondaire 7 peuvent avantageusement être réalisés en une seule et même matière. De préférence, les composants primaire 4 et secondaire 7 sont ainsi tous deux en matériau polymère, par exemple en polyétheréthercétone PEEK. Un tel matériau présente en effet l'intérêt d'être biocompatible, radio-transparent, léger et peu coûteux à mettre en oeuvre. Alternativement, les composants primaire 4 et secondaire 6 pourront être réalisés dans un autre matériau, par exemple un matériau métallique de type inox ou titane.

Selon l'invention, le connecteur femelle 9 comprend un corps principal 10 qui s'étend longitudinalement entre une extrémité arrière 11 reliée au corps d'ancrage secondaire 8 et une face avant 12 opposée, laquelle est préférentiellement délimitée par un bord avant 13 périphérique. De préférence, le corps principal 10 s'étend longitudinalement le long du quatrième axe D-D' d'extension du connecteur femelle 9. Le corps principal 10 présente une paroi latérale 14, qui relie ladite extrémité arrière 11 et ladite face avant 12. De préférence, cette paroi latérale 14 est cylindrique ou de forme généralement convergente (par exemple conique), depuis la face avant 12 en direction de l'extrémité arrière 11, le long du quatrième axe D-D'. De préférence, la face avant 12 s'étend dans un plan sensiblement orthogonal au quatrième axe D-D'. On entend par *« forme générale* » la forme extérieure globale de l'élément concerné, à l'exclusion d'éventuels méplats, chanfreins, dents, et autres éléments saillants ou rentrants. Tel qu'illustré aux figures 4 à 6, le connecteur femelle 9 comprend un logement 15, qui est ménagé au sein dudit corps principal 10 et qui est configuré pour recevoir le connecteur mâle 8 afin de relier ensemble (c'est-à-dire afin de connecter, d'accoupler ensemble) les composants primaire 4 et secondaire 7. Le logement 15 forme avantageusement un espace creux, défini par une paroi intérieure 16, à l'intérieur du corps principal 10, et de formes et dimension conjuguées à celles respectives du connecteur mâle 6. Le logement 15 est ainsi avantageusement configuré et dimensionné pour recevoir en son sein au moins une partie, et de préférence l'intégralité du connecteur mâle 6. De préférence, le logement 15 s'étend longitudinalement selon une direction parallèle, et de manière plus préférentielle confondue, avec le quatrième axe D-D' d'extension du connecteur femelle 9. Préférentiellement, le logement 15 s'étend depuis la face avant 12 jusqu'à une paroi de fond 17 opposée, laquelle est avantageusement positionnée au voisinage immédiat de l'extrémité arrière 11 du corps principal 10. De préférence encore, le logement 15 comporte deux parties distinctes le long du quatrième axe D-D', en l'espèce une première partie 15A qui s'étend depuis ladite face avant 12, et une deuxième partie 15B qui prolonge la première partie 15A jusqu'à ladite paroi de fond 17 (figure 6).

Selon l'invention, le connecteur femelle 9 comprend en outre une ouverture 18 qui est ménagée dans ledit corps principal 10, laquelle ouverture 18 est en communication (avantageusement directe) avec ledit logement 15, c'est-à-dire qu'elle donne accès à ce dernier depuis le milieu extérieur au corps principal 10. Selon l'invention, l'ouverture 18 s'étend de manière continue à la fois sur ladite face avant 12 et ladite paroi latérale 14. Avantageusement, l'ouverture 18 du connecteur femelle 9 comprend une première zone 18A d'ouverture, qui s'étend sur ladite face avant 12, et une deuxième zone 18B d'ouverture, laquelle est contigue à la première zone 18A, prolonge immédiatement cette dernière et s'étend sur au moins une partie de ladite paroi latérale 14. L'ouverture 18 est, de manière générale, dimensionnée et configurée pour autoriser une introduction latérale du connecteur mâle 6 (ou d'au moins une partie de ce dernier) dans ledit logement 15 par ladite ouverture 18. Cela signifie en particulier, que la configuration, la conformation et les dimensions de la deuxième zone 18B de l'ouverture 18 sont avantageusement choisies en considération des configuration, conformation et dimensions propres du connecteur mâle 8, de sorte qu'une portion au moins (par exemple une portion libre extrémale) de ce dernier puisse être introduite dans ledit logement 15 par l'intermédiaire de ladite deuxième zone 18B d'ouverture. Avantageusement, le logement 15 débouche du corps principal 10 au niveau de la face avant 12 de ce dernier par l'intermédiaire de ladite première zone 18A d'ouverture, et cette dernière est préférentiellement dimensionnée, conformée et configurée pour autoriser une introduction faciale du connecteur mâle 6, et de préférence de l'intégralité de ce dernier, dans ledit logement 15 par l'intermédiaire de la première zone 18A. Le connecteur femelle 9 est ainsi avantageusement apte à recevoir, au moins en partie, le connecteur mâle 6 au sein de son logement 15 par l'intermédiaire à la fois de la première zone 18A et de la deuxième zone 18B de son ouverture 18.

De préférence, l'introduction du connecteur mâle 6 dans le logement 15 par l'intermédiaire de la seule deuxième zone 18B de l'ouverture 18 ne permet pas de relier entre eux les composants primaire 4 et secondaire 7 de manière pleine et satisfaisante. En effet, lorsque les composants primaire 4 et secondaire 7 sont reliés ensemble, ledit connecteur mâle 6 est préférentiellement logé, au moins en partie, dans le logement 15 de sorte que son deuxième axe B-B' d'extension soit confondu avec le quatrième axe D-D'. En revanche, il reste avantageusement possible d'obtenir une pleine coopération des connecteurs mâle 6 et femelle 9 par introduction d'au moins une partie du connecteur mâle 6 dans ledit logement 15 par le seul intermédiaire de la première zone 18A de l'ouverture 18.

La conformation, la configuration et le dimensionnement de ce connecteur femelle 9, et en particulier de son logement 15 et de son ouverture 18, seront mieux compris en relation avec la description qui suit du connecteur mâle 6. De préférence, le connecteur mâle 6 se présente sous la forme d'un corps allongé, et s'étend préférentiellement longitudinalement entre une première extrémité 19 reliée (directement ou indirectement) au corps d'ancrage primaire 5 et une deuxième extrémité 20 libre opposée, le long du deuxième axe B-B' (figures 2 et 3). La deuxième extrémité libre 20 du connecteur mâle 6 est avantageusement chanfreinée pour faciliter son introduction au sein du logement 15 du connecteur femelle 9. La première extrémité 19 du connecteur mâle 6 forme quant à elle préférentiellement un point d'attache du connecteur mâle 6 avec le corps d'ancrage primaire 5. De préférence, le connecteur mâle 6 présente une surface externe 21, qui relie la première extrémité 19 à la deuxième extrémité 20 libre du connecteur male 6, et forme par exemple un corps sensiblement plein et massif, délimité par ladite surface externe 21. Tel qu'illustré, cette dernière est préférentiellement de forme cylindrique à base circulaire. Le connecteur male 6 se présente ainsi avantageusement sous la forme d'une tige cylindrique de section (orthogonale audit deuxième axe B-B') sensiblement circulaire. Néanmoins, d'autres formes pourront être envisagées et ladite surface externe 21 pourra, par exemple, être de forme cylindrique à base polygonale (par exemple à base hexagonale). Alternativement encore, la surface externe 21 pourra affecter une forme généralement convergente (par exemple conique) en direction de la deuxième extrémité 20 libre du connecteur male 6. Avantageusement, le connecteur mâle 6 comprend une première portion 22A et une deuxième portion 22B, la première portion 22A s'étendant depuis la première extrémité 19, tandis que la deuxième portion 22B prolonge la première portion 22A jusqu'à ladite deuxième extrémité 20 libre. La deuxième portion 22B forme ainsi une portion extrémale (ou distale) du connecteur mâle 6, avantageusement destinée à être introduite au sein du logement 15 du connecteur femelle 9 avant la première portion 22A. Compte-tenu de ce qui précède, ces première 22A et deuxième 22B portions peuvent présenter une section moyenne identique ou non. Avantageusement, la première portion 22A est prévue pour être reçue, au moins en partie, dans la première partie 15A dudit logement 15, tandis que la deuxième portion 22B est prévue pour être reçue, au moins en partie, dans la deuxième partie 15B dudit logement 15, lorsque les composants primaire 4 et secondaire 7 sont reliés ensemble.

En conséquence, le logement 15 du connecteur femelle 9 se présente alors préférentiellement sous la forme d'un espace creux défini par une paroi intérieure 16 cylindrique, et de forme complémentaire à la forme générale cylindrique à base circulaire de la surface externe 21 du connecteur mâle 6. Compte-tenu de ce qui précède, les première 15A et deuxième 15B parties du logement 15 peuvent présenter une section moyenne identique ou non. De la sorte, le connecteur mâle 6 peut venir se loger étroitement (éventuellement avec un très léger jeu pour faciliter l'assemblage relatif des composants primaire 4 et secondaire 7) dans ledit logement 15. De préférence encore, le logement 15 s'étend longitudinalement sur une longueur sensiblement voisine de la longueur du connecteur mâle 8 (mesurée depuis la première extrémité 19 jusqu'à la deuxième extrémité 20 libre de ce dernier). Cependant, comme on le verra plus loin, il est optionnellement envisageable que le logement 15 s'étende longitudinalement sur une longueur inférieure à la longueur du connecteur mâle 6.

De manière préférentielle, la deuxième zone 18B de l'ouverture 18 est conformée, configurée et dimensionnée pour autoriser une introduction latérale de la deuxième portion 22B du connecteur mâle 6 dans le logement 15 par la deuxième zone 18B de l'ouverture 18. La deuxième zone 18B se présente alors, de préférence, tel qu'illustré aux figures, sous la forme d'une fenêtre sensiblement rectangulaire, et s'étend longitudinalement, depuis le bord avant 13 et en direction de l'extrémité arrière 11 dudit corps principal 10, selon une direction sensiblement parallèle au quatrième axe D-D' d'extension de ce dernier. Autrement dit, la deuxième zone 18B de l'ouverture 18 s'étend de préférence axialement en regard du logement 15, sur au moins une partie de la longueur de ce dernier. Avantageusement, la deuxième zone 18B s'étend longitudinalement sur une longueur L1 du corps principal 10 sensiblement égale à (ou voisine de) la longueur L2 de la deuxième portion 22B du connecteur mâle 6, et en tout état de cause sur une longueur L1 préférentiellement inférieure à la longueur totale du connecteur mâle 6. De préférence, la deuxième zone 18B de l'ouverture 18 s'étend transversalement sur une largeur ℓ1 au moins égale (et de préférence très légèrement supérieure) à l'encombrement transversal maximal ℓ2 de la deuxième portion 22B du connecteur mâle 6. Cela signifie, en particulier, que la largeur ℓ1 (de préférence considérée selon une direction orthogonale au quatrième axe D-D') de la deuxième zone 18B est au moins égale (et de préférence très légèrement supérieure) au diamètre de la deuxième portion 22B du connecteur mâle 6 (lorsque ce dernier est de forme cylindrique à base circulaire, ou au diamètre du cercle dans lequel s'inscrit la section polygonale du connecteur mâle 6, lorsque la surface externe 21 de ce dernier est de forme générale cylindrique à base polygonale), Avantageusement, les bords respectifs des première 18A et deuxième 18B zones de l'ouverture 18 sont chanfreinés ou évasés pour faciliter l'introduction du connecteur mâle 6, en particulier de sa deuxième portion 22B, dans le logement 15 du connecteur femelle 9 (figures 4 à 6).

Compte tenu de ce qui précède, on comprend que le connecteur mâle 6 peut être avantageusement introduit dans le logement 15 du connecteur femelle 9 par le biais d'une combinaison d'un déplacement selon une direction sensiblement orthogonale aux deuxième B-B' et quatrième D-D' axes d'extension longitudinale respectifs des connecteurs mâle 6 et femelle 9, et d'un déplacement selon une direction sensiblement confondue à ces mêmes deuxième B-B' et quatrième D-D' axes. Ainsi, les corps d'ancrage primaire 5 et secondaire 8 étant respectivement ancrés dans le premier os 2 et dans le deuxième os 3, une partie au moins du connecteur mâle 8 (en l'espèce au moins sa deuxième portion 22A extrémale) peut être introduite latéralement dans le logement 15 du connecteur femelle 9, sans qu'il soit nécessaire au préalable d'écarter les premier 2 et deuxième 3 os d'une distance au moins égale à la longueur totale du connecteur mâle 8 selon le deuxième axe B-B'. Par conséquent, le chirurgien qui met en oeuvre l'implant 1 de l'invention dans le corps d'un patient pourra avantageusement ancrer l'un puis l'autre desdits composants primaire 4 et secondaire 7 respectivement dans les premier 2 et deuxième 3 os, puis relier entre eux les composants primaire 4 et secondaire 7 par l'intermédiaire de leurs connecteurs mâle 6 et femelle 9 respectifs, tout en conservant une distance entre les premier 2 et deuxième 3 os particulièrement restreinte. La distraction interarticulaire nécessaire à la mise en oeuvre de l'implant 1 de l'invention est ainsi particulièrement limitée, ce qui permet avantageusement de limiter les effets délétères de l'opération sur les tendons et autres tissus mous environnants.

De préférence, les connecteurs mâle 6 et femelle 9 comprennent respectivement un premier et un deuxième moyens de verrouillage complémentaires qui coopèrent pour assurer le verrouillage relatif des composants primaire 4 et secondaire 7, une fois ces derniers reliés, c'est-à-dire une fois le connecteur mâle 6 introduit au sein du logement 15 du connecteur femelle 9 (de préférence de manière coaxiale audit logement 15). Autrement dit, le connecteur mâle 6 porte un premier moyen de verrouillage, par exemple au niveau de sa surface externe 21 ou de sa deuxième extrémité 21 libre, conçu pour coopérer avec un deuxième moyen de verrouillage correspondant porté par le connecteur femelle 9, par exemple au niveau de la paroi interne 16 de son logement 15, pour d'éviter, ou au moins de limiter fortement, tout risque de dissociation intempestive des composants primaire 4 et secondaire 6, une fois ces derniers reliés ensemble. En particulier, ces moyens de verrouillage complémentaires sont avantageusement conçus pour interdire, ou au moins limiter fortement, tout déplacement relatif des composants primaire 4 et secondaire 7 selon une direction parallèle aux deuxième B-B' et quatrième D-D' axes d'extension respectifs des connecteurs mâle 6 et femelle 9, une fois les composants primaire 4 et secondaire 6 reliés ensemble par l'intermédiaire de ces derniers.

Lesdits premier et deuxième moyens de verrouillage peuvent être de tout type connu de l'art antérieur, et être conçus assurer un verrouillage relatif des composants primaire 4 et secondaire 6 par exemple par collage, vissage, clipsage, encliquetage ou encore par emmanchement à force. De préférence, tel qu'illustré aux figures, le verrouillage est assuré par encliquetage, les premier et deuxième moyens de verrouillage étant par exemple respectivement formés par au moins une dent 23 qui fait saillie de la surface externe 21 du connecteur mâle 6, et par au moins au moins un cran 24 complémentaire ménagé dans la paroi interne 16 du logement 15 du connecteur femelle 9. La coopération de la dent 23 et du cran 24 pourra alors être avantageusement favorisée par déformation élastique de la dent 23, et/ ou du cran 24, et / ou encore de la paroi latérale 14 du corps principal 10 du connecteur femelle 9.

Selon une variante préférentielle, l'implant 1 est avantageusement prévu pour être laissé dans le corps du patient à l'issue de l'opération, tout au long de la vie dudit patient. Dans ce cas, les premier et deuxième moyens de verrouillage sont avantageusement conçus et configurés pour assurer un verrouillage relatif définitif (ou au moins difficilement réversible sans exercer d'efforts conséquents) des composants primaire 4 et secondaire 7. De préférence, le premier moyen de verrouillage est alors formé par au moins une dent 23 (ou ergot, ou encore bosse) qui fait saillie de la surface externe 21 du connecteur mâle 8 selon tout le pourtour de la section de ce dernier, tandis le deuxième moyen de verrouillage pourra être formé par au moins un cran 24 (ou entaille, ou encore renfoncement) complémentaire ménagé dans la paroi interne 16 du logement 15 du connecteur femelle 9 selon tout le pourtour de la section dudit logement 15. Avantageusement, la dent 23 et le cran 24 correspondant pourront présenter chacun une face inclinée et une face droite opposée conjuguées, la coopération des faces inclinées favorisant l'encliquetage de la dent 23 dans le cran 24, tandis que la coopération des faces droites s'oppose, une fois la dent 23 encliquetée dans le cran 24, au désencliquetage relatif de ces derniers. Alternativement, lesdits moyens de verrouillage pourront être formés ou remplacés par une colle introduite dans le logement 15, qui assure une adhésion forte entre la surface externe 21 du connecteur mâle 6 et la paroi interne 16 du logement 15 du connecteur femelle 9.

Selon une autre variante préférentielle, l'implant 1 pourra au contraire être prévu pour autoriser son ablation future, c'est-à-dire pour autoriser son extraction totale ou partielle du corps du patient (extraction de l'un et / ou de l'autre des composants primaire 4 et secondaire 7), ou au moins pour autoriser la déconnexion et la séparation des composants primaire 4 et secondaire 7. A cet effet, les premier et deuxième moyens de verrouillage complémentaires seront préférentiellement conçus et configurés pour assurer un verrouillage relatif réversible des composants primaire 4 et secondaire 7, de sorte que le déverrouillage et la séparation de ces derniers sont rendus possibles. De tels moyens de verrouillage réversible pourront, par exemple, être respectivement formés par au moins une dent 23 (ou ergot, ou encore bosse) qui fait saillie ponctuellement de la surface externe 21 du connecteur mâle 6, et par au moins au moins un cran 24 (ou entaille, ou encore renfoncement) complémentaire ménagé ponctuellement dans la paroi interne 16 du logement 15 du connecteur femelle 9. Ainsi, une légère rotation du connecteur mâle 6 dans le connecteur femelle 9 selon le deuxième axe B-B' pourra suffire à désengager ladite dent 23 et ledit cran 24 et à autoriser le découplage des composants primaire 4 et secondaire 7.

De préférence, les premier et deuxième moyens de verrouillage sont plus spécifiquement conçus et configurés pour autoriser un déverrouillage relatif des composants primaire 4 et secondaire 7 par rotation d'un quart de tour du connecteur mâle 6 dans le connecteur femelle 9 selon le deuxième axe B-B' d'extension du connecteur mâle 6. Ainsi, il est possible d'obtenir un verrouillage réversible et efficace, en évitant tout risque de déverrouillage intempestif des connecteurs mâle 6 et femelle 9, en particulier sous l'effet d'une légère mise en rotation relative involontaire des premier 2 et deuxième 3 os. Par exemple, tel qu'illustré aux figures, le premier moyen de verrouillage pourra avantageusement être formé d'une part, d'au moins une dent 23 (ou ergot, ou encore bosse) qui fait saillie de la surface externe 21 du connecteur mâle 6 selon tout le pourtour de la section de ce dernier, et d'autre part, de deux méplats 25 ménagés axialement, selon le deuxième axe B-B' d'extension du connecteur mâle 6, dans et à travers la dent 23 et positionnés parallèlement l'un à l'autre, de part et d'autre du deuxième axe B-B'. Le deuxième moyen de verrouillage pourra quant à lui être avantageusement formé d'une part, par au moins au moins un cran 24 (ou entaille, ou encore renfoncement) complémentaire de ladite dent 23, et ménagé dans la paroi interne 16 du logement 15 du connecteur femelle 9 selon tout le pourtour de la section du logement 15, et d'autre part, de deux rainures 26 de dimensions conjuguées à celles des méplats 25 du premier moyen de verrouillage, ces rainures 26 étant ménagées axialement, selon le quatrième axe D-D' d'extension du connecteur femelle 9 et de son corps principal 10, dans et à travers le cran 24 et étant positionnées parallèlement l'une à l'autre de part et d'autre du quatrième axe D-D'. Ainsi, une rotation d'un quart de tour du connecteur mâle 6 dans le connecteur femelle 9 selon le deuxième axe B-B' d'extension du connecteur mâle 6 entraînera avantageusement la mise en correspondance des méplats 25 et rainures 26 correspondantes, autorisant ainsi la libération de la dent 23 du cran 24 correspondant. Avantageusement, la dent 23 et le cran 24 pourront respectivement présenter une face inclinée et une face droite opposée, comme évoqué précédemment, de sorte à obtenir un verrouillage réversible, robuste et fiable. De préférence, au moins l'une des rainures 26 pourra être alignée et centrée sur la deuxième zone 18B de l'ouverture 18 du connecteur femelle 9. Dans ce cas, la rainure 26 considérée sera avantageusement formée, sur au moins une partie de sa longueur, par ladite deuxième zone 18B.

De manière préférentielle, et tel qu'illustré aux figures, les premier et deuxième moyens de verrouillage complémentaires sont respectivement formés d'une pluralité de dents 23 (ou d'ergots, ou encore de bosses) alignées selon le deuxième axe B-B' d'extension du connecteur mâle 6, et d'une pluralité de crans 24 (ou d'entailles, ou encore de renfoncements) alignés selon le quatrième axe D-D' d'extension du connecteur femelle 9 et de son corps principal 10. Ainsi, les premier et deuxième moyens de verrouillage forment avantageusement un système de dents 23 et de crans 24 étagés (ou d'au moins un cran 24) complémentaires autorisant un ajustement progressif de l'écartement relatif des corps d'ancrage primaire 5 et secondaire 8. En effet, le connecteur mâle 6 étant introduit au sein du logement 15 du connecteur femelle 9, de préférence de sorte à ce que le connecteur mâle 6 s'étende de manière coaxiale avec le logement 15, une translation axiale du connecteur mâle 6 dans le logement 15 permet alors avantageusement de mettre en prise les moyens de verrouillage, et de verrouiller ainsi les composants primaire 4 et secondaire 7, selon une pluralité de positions axiales, lesquelles correspondent à un écartement plus ou moins important des corps d'ancrage primaire 5 et secondaire 8 auxquels sont respectivement reliés les connecteurs mâle 6 et femelle 9. Ainsi, après avoir introduit et ancré les composants primaire 4 et secondaire 7 respectivement dans le premier os 2 et dans le deuxième os 3, le chirurgien pourra avantageusement relier ensemble les composants primaire 4 et secondaire 7 et les verrouillés l'un à l'autre selon une première position correspondant à un premier écartement relatif de leurs corps d'ancrage respectifs, puis rapprocher progressivement les premier 2 et deuxième 3 os, de préférence jusqu'à venir les abouter, tout en conservant le verrouillage relatif des composants primaire 4 et secondaire 7.

Tel qu'illustré aux figures 4 à 6, ledit connecteur femelle 9 pourra optionnellement et avantageusement comprendre une fente 27 ménagée dans la paroi latérale 14 du corps principal 10 du connecteur femelle 9, en communication avec le logement 15 de ce dernier. De préférence, cette fente 27 traversante est positionnée en regard de l'ouverture 18 et, plus précisément encore, en regard de la deuxième zone 18B de cette dernière. La fente 27 s'étend avantageusement axialement selon le quatrième axe D-D', depuis la face avant 12 (de laquelle elle débouche préférentiellement) en direction de l'extrémité arrière 11 du corps principal 10, et de préférence parallèlement à la deuxième zone 18B de l'ouverture 18. De manière préférentielle, la fente 27 s'étend sur une partie au moins de la longueur dudit corps principal 10 et, de manière plus préférentielle encore, sur une longueur sensiblement voisine de la longueur L1 de ladite deuxième zone 18B. Une telle fente 27 permet ainsi avantageusement de faciliter une déformation, de préférence élastique, du corps principal 10 (et en particulier de sa de la paroi latérale 14) lors du verrouillage relatif des composants primaire 4 et secondaire 7, et / ou lors de l'ajustement de l'écartement relatif des corps d'ancrage primaire 5 et secondaire 8 respectifs de ces derniers. Il est alors en particulier possible d'ajuster manuellement, avec un effort suffisant mais toutefois mesuré, l'écartement relatif des corps d'ancrage primaire 5 et secondaire 8, et donc des premier 2 et deuxième 3 os que l'on souhaite fusionner ensemble.

De manière avantageuse, et tels que cela apparaît aux figures 2 et 3 et 10 à 13, le premier moyen de verrouillage est porté par la première portion 22A du connecteur mâle 6 uniquement. La deuxième portion 22B extrémale du connecteur mâle 6 est ainsi avantageusement dépourvue de tout premier moyen de verrouillage. De la sorte, il est possible d'introduire la deuxième portion 22B du connecteur mâle 6 dans le logement 15 par le biais de l'ouverture 18, et de relier ainsi les composants primaire 4 et secondaire 7 sans toutefois les verrouiller l'un à l'autre. Le verrouillage relatif des composants primaire 4 et secondaire 7 ne sera alors possible que lorsque la première portion 22A du connecteur mâle 6 sera à son tour introduite dans le logement 15 du connecteur femelle 9. Une telle conception s'avère pratique et intéressante, en particulier dans le cas où les moyens de verrouillage complémentaires ne sont pas conçus pour assurer un verrouillage relatif réversible des composants primaire 4 et secondaire 7, puisque le chirurgien pourra alors réaliser des essais d'assemblage relatif partiel de ces derniers, sans risquer pour autant de les verrouiller immédiatement et de manière irréversible. Par ailleurs, dans un tel cas, le deuxième moyen de verrouillage correspondant pourra avantageusement être respectivement porté uniquement ou majoritairement par la première partie 15A du logement 15, ce qui permettra de faciliter la conception et la fabrication du composant secondaire 7.

De préférence, les connecteurs mâle 6 et femelle 9 comprennent respectivement un premier et un deuxième moyen de blocage complémentaires qui coopèrent pour limiter ou empêcher la rotation du connecteur mâle 6 relativement au connecteur femelle 9, lorsque le connecteur mâle 6 est en place dans le logement 15 du connecteur femelle 9, et en particulier dans le cas envisagé ci-avant où le connecteur mâle 8 et le logement 15 sont de forme générale cylindrique à base circulaire. Lorsque l'implant 1 est mis en place au niveau des premier 2 et deuxième 3 os, de tels moyens de blocage contribuent ainsi avantageusement à limiter ou à empêcher la rotation du premier os 2 et du deuxième os 3 l'un par rapport à l'autre. Ainsi, l'implant 1 permet avantageusement d'immobiliser ces derniers à la fois en translation et en rotation l'un par rapport à l'autre, ce qui favorise leur fusion osseuse et autorise donc une arthrodèse rapide. Par exemple, de tels premier et deuxième moyens de blocage complémentaires pourront être respectivement formés de premier 28 et deuxième 29 méplats axiaux longitudinaux, ménagés respectivement dans la surface externe 21 du connecteur mâle 6 et dans la paroi interne 16 du logement 15, de préférence au niveau uniquement de la deuxième portion 22B du connecteur mâle 6 et de la deuxième partie 15B du logement 15 du connecteur femelle 9. Eventuellement, de tels moyens de blocage en rotation pourront faire office de système détrompeur, qui limite les configurations possibles de coopération des connecteurs mâle 6 et femelle 9 (en particulier dans le cas où le composant primaire 4 est coudé, comme décrit plus loin).

De préférence, le connecteur femelle 9 sera pourvu d'au moins un méplat 30 axial longitudinal, et de préférence de deux méplats 30 axiaux longitudinaux, ménagés dans sa paroi latérale 14, et qui s'étendent de préférence depuis le bord avant 13 jusqu'à l'extrémité arrière 11 de son corps principal 10 selon le quatrième axe D-D' (figures 4 et 5). Avantageusement, ces méplats 30 sont positionnés parallèlement l'un à l'autre de part et d'autre du quatrième axe D-D'. Le connecteur femelle 9 étant préférentiellement destiné à être introduit dans le deuxième os 3, de tels méplats 30 contribuent avantageusement à bloquer le connecteur femelle 9 en rotation dans le deuxième os 3 selon le quatrième axe D-D'. De manière plus avantageuse encore, l'un 34 desdits méplats 30 est aligné et centré longitudinalement avec la deuxième zone 18B de l'ouverture 18. L'autre desdits méplats 30 est durant à lui avantageusement aligné et centré longitudinalement sur la fente 27 optionnelle. Tel que cela ressort particulièrement de la figure 5, le corps principal 10 du connecteur femelle 9 présente alors de préférence une section (orthogonale audit quatrième axe D-D') avec deux côtés opposés sensiblement rectilignes et parallèles, les deux autres côtés étant sensiblement curvilignes. De manière particulièrement préférentielle, le corps principal 10 comprend ainsi une première face 31 dite dorsale, portant la deuxième zone 18B de l'ouverture 18 et l'un des méplats 30, une deuxième face 32 opposée dite ventrale, portant ladite fente 27 optionnelle et l'autre des méplats 30, et des troisième 33 et quatrième 34 faces latérales, opposées l'une à l'autre et reliant lesdites faces dorsale 31 et ventrale 32.

Ci-après vont être maintenant décrits en détails les corps d'ancrage primaire 5 et secondaire 8 respectifs des composants primaire 4 et secondaire 7 de l'implant 1 de l'invention. Bien évidemment, la description qui suit ne présente pas de caractère limitatif, et d'autres modes de réalisations et variantes que ceux exposés ci-après pourront être envisagés sans pour autant sortir du cadre de l'invention. De préférence, le corps d'ancrage primaire 5 est prévu pour être intégralement inséré dans le premier os 2, tandis que le connecteur mâle 6 est prévu pour faire saillie hors du premier os 2, lorsque le composant primaire 4 est en place dans ce dernier. Respectivement, le corps d'ancrage secondaire 8 est préférentiellement prévu pour être intégralement inséré dans le deuxième os 3, le connecteur femelle 9 étant avantageusement lui aussi prévu pour être intégralement inséré dans ce dernier (et donc pour ne pas faire saillie hors du deuxième os 3), lorsque le composant secondaire 7 est en place dans le deuxième os 3 (figures 7 à 9 et 15 à 17).

De préférence, les corps d'ancrage primaire 5 et secondaire 8 sont respectivement conçus pour assurer une insertion et un ancrage du composant primaire 4 dans le premier os 2, et du composant secondaire 7 dans le deuxième os 3, par impaction ou par coincement en force, de sorte que lesdits composants 4, 7 peuvent être de préférence insérés et ancrés dans les os 2, 3 correspondants selon un mouvement de translation pure. A ce titre, lesdits corps d'ancrage primaire 5 et secondaire 7 sont alors de préférence respectivement dépourvus de moyen de vissage du corps d'ancrage primaire 5 dans le premier os 2 et du corps d'ancrage secondaire 8 dans le deuxième os 3. Une telle conception est particulièrement avantageuse, notamment dans le cas où les composants primaire 4 et secondaire 6 forment chacun une pièce monobloc d'un seul tenant. En effet, l'insertion et l'ancrage du composant primaire 4 dans le premier os 2, et du composant secondaire 7 dans le deuxième os 3 par impaction ou par coincement en force permettent avantageusement d'assurer une parfaite maîtrise de l'orientation finale des composants primaire 4 et secondaire 7. En particulier, cela permet d'assurer une parfaite maîtrise de l'orientation relative des connecteurs mâle 6 et femelle 9 respectifs des composants primaire 4 et secondaire 7, respectivement autour des deuxième B-B' et quatrième D-D' axes d'extension longitudinale, lors de l'implantation de ces derniers dans le corps du patient. On facilite donc ainsi avantageusement une parfaite mise en regard du connecteur mâle 6 avec l'ouverture 18 du connecteur femelle 9, et en particulier avec la deuxième zone 18B d'ouverture de ladite ouverture 18. L'assemblage relatif des composants primaire 4 et secondaire 7, une fois ces derniers implantés dans le corps du patient, en est ainsi avantageusement facilité. De préférence, le corps d'ancrage primaire 5 est destiné à être inséré et ancré dans un canal médullaire du premier os 2, tandis que le corps d'ancrage secondaire 8 est respectivement destiné à être inséré et ancré dans un canal médullaire du deuxième os 3. Les corps d'ancrage primaire 5 et secondaire 8 peuvent dans ce cas être implantés dans les canaux médullaires du premier 2 et du deuxième os 3, par exemple après que l'extrémité de l'os concerné (formée par exemple par une surface cartilagineuse extrémale) ait été percée ou retirée afin de ménager une ouverture d'accès audit canal médullaire. Le stock osseux intérieur desdits canaux médullaires doit également préférentiellement être aménagé pour permettre l'accueil du corps d'ancrage primaire 5, respectivement du corps d'ancrage secondaire 8. Bien entendu, sans sortir du cadre de l'invention, le corps d'ancrage primaire 5 et / ou le corps d'ancrage secondaire 8 pourront être conçus pour être ancrés dans toute autre partie desdits premier 2 et deuxième 3 os, et par exemple pour être ancrés dans un orifice préalablement ménagé dans la partie corticale de ces derniers. Dans le cas préférentiel où l'implant 1 forme préférentiellement un implant intra-médullaire, les corps d'ancrage primaire 5 et secondaire 8 sont avantageusement conformés et configurés pour pouvoir être insérés axialement dans les canaux médullaires respectifs des premier 2 et deuxième 3 os. En particulier, le canal médullaire de tels os étant de forme générale tubulaire, sensiblement cylindrique, les corps d'ancrage primaire 5 et secondaire 8 ont préférentiellement une forme générale allongée et axiale, et de préférence cylindrique, conique, ou encore prismatique, et seront conformés pour être insérés de façon coaxiale au sein du canal médullaire concerné (figures 7 à 9 et 15 à 17).

D'une manière générale, le corps d'ancrage primaire 5 s'étend de préférence longitudinalement le long du premier axe A-A' entre une extrémité de pénétration primaire 35 dans le premier os 2 et une extrémité de base primaire 36 opposée. L'extrémité de pénétration primaire 35 forme une première extrémité libre de l'implant 1, tandis que l'extrémité de base primaire 36 opposée forme quant à elle préférentiellement un point d'attache du corps d'ancrage primaire 5 avec le connecteur mâle 6. Respectivement, le corps d'ancrage secondaire 8 s'étend longitudinalement le long du deuxième axe B-B' entre une extrémité de pénétration secondaire 37 dans le deuxième os 3 et une extrémité de base secondaire 38 opposée. L'extrémité de pénétration secondaire 37 forme une deuxième extrémité libre de l'implant 1, tandis que l'extrémité de base secondaire 38 opposée forme quant à elle préférentiellement un point d'attache du corps d'ancrage secondaire 8 avec le connecteur femelle 9.

Selon un premier mode de réalisation préférentiel de l'implant 1 de l'invention (figures 1 à 9), le corps d'ancrage primaire 5 présente une surface externe primaire 39, qui relie l'extrémité de base primaire 36 à l'extrémité de pénétration primaire 35, et forme par exemple un corps sensiblement plein et massif, délimité par ladite surface externe primaire 39. De préférence, l'extrémité de pénétration primaire 35 est effilée, conique, bombée ou chanfreinée pour faciliter son insertion et sa pénétration dans le premier os 2. Tel qu'illustré, la surface externe primaire 39 est préférentiellement de forme généralement convergente (par exemple conique), en direction de l'extrémité de pénétration primaire 35, le long du premier axe A-A'. Une telle conformation permet notamment de favoriser le coincement (par exemple en force) du corps d'ancrage primaire 5 dans le premier os 2, le corps d'ancrage primaire 5 pouvant ainsi par exemple s'adapter à des canaux médullaires de diamètres. De préférence, le corps d'ancrage primaire 5 est pourvu d'au moins un méplat primaire 40 longitudinal ménagé dans la surface externe primaire 41. Avantageusement, ce méplat primaire 40 s'étend selon le premier axe A-A', de préférence depuis ladite extrémité de pénétration primaire 40 jusqu'à ladite extrémité de base primaire 41 opposée (figures 1 à 3). De manière encore plus préférentielle, le corps d'ancrage primaire 5 est pourvu de deux méplats primaires 40 longitudinaux ménagés dans la surface externe primaire 39. Ces méplats primaires 40 s'étendent alors préférentiellement tous deux selon le premier axe A-A', depuis ladite extrémité de pénétration primaire 35 jusqu'à ladite extrémité de base primaire 36 opposée. Avantageusement de formes et dimensions identiques, les méplats primaires 40 sont préférentiellement positionnés parallèlement l'un à l'autre de part et d'autre du premier axe A-A'. Tel qu'illustré, le corps d'ancrage primaire 5 peut ainsi avantageusement se présenter sous la forme d'un corps ou d'une plaque de profil sensiblement trapézoïdal, et de section sensiblement rectangulaire ou trapézoïdale. La présence d'un tel méplat primaire 40 (et de préférence de deux méplat primaire 40) favorise avantageusement une bonne adaptation à l'anatomie osseuse du patient, tout en contribuant préférentiellement à empêcher la mise en rotation du composant primaire 4, une fois ce dernier ancré dans le canal médullaire du premier os 2.

De manière avantageusement similaire, le corps d'ancrage secondaire 8 présente de préférence une surface externe secondaire 41, qui relie l'extrémité de base secondaire 38 à l'extrémité de pénétration secondaire 37, et forme par exemple un corps sensiblement plein et massif, délimité par ladite surface externe secondaire 41 (figures 4 et 5). Tel qu'illustré, la surface externe secondaire 41 est préférentiellement de forme généralement convergente (par exemple conique), en direction de l'extrémité de pénétration secondaire 37, le long du deuxième axe B-B'. Avantageusement, lorsque le corps d'ancrage secondaire 8 et le connecteur femelle 9 forment une pièce monobloc d'un seul tenant, la surface externe secondaire 41 du corps d'ancrage secondaire 8 prolonge avantageusement la paroi latérale 14 du connecteur femelle 9.

De préférence, le corps d'ancrage secondaire 8 est pourvu d'au moins un méplat secondaire 42 longitudinal ménagé dans ladite surface externe secondaire 41 du corps d'ancrage secondaire 8. Avantageusement, tel qu'illustré aux figures 4 et 5, ledit méplat secondaire 42 s'étend selon le deuxième axe B-B', de préférence depuis ladite extrémité de pénétration secondaire 37 jusqu'à ladite extrémité de base secondaire 38 opposée. De manière encore plus préférentielle, le corps d'ancrage secondaire 7 est pourvu de deux méplats secondaires 42 longitudinaux ménagés dans ladite surface externe secondaire 41 (figure 6). Ces méplats secondaires 42 s'étendent alors préférentiellement tous deux selon le deuxième axe B-B', depuis ladite extrémité de pénétration secondaire 37 jusqu'à ladite extrémité de base secondaire 38 opposée. Avantageusement de formes et dimensions identiques, ces méplats secondaire 42 sont préférentiellement positionnés parallèlement l'un à l'autre de part et d'autre du deuxième axe B-B'. Tel qu'illustré, le corps d'ancrage secondaire 8 peut ainsi avantageusement se présenter sous la forme d'un corps de profil sensiblement trapézoïdal, et de section sensiblement rectangulaire ou trapézoïdale. La présence d'un tel méplat secondaire 42 (et de préférence de deux méplats secondaires 42) favorise avantageusement une bonne adaptation à l'anatomie osseuse du patient, tout en contribuant préférentiellement à empêcher la mise en rotation du composant secondaire 8, une fois ce dernier ancré dans le canal médullaire du deuxième os 3. Avantageusement, le ou les méplats d'ancrage secondaire 42 sont agencés de manière à prolonger axialement (ou au moins à être aligné axialement avec) le ou les méplats 30 respectifs du corps principal 10 du connecteur femelle 9 (figures 4 et 5).

Selon ce premier mode de réalisation préférentiel de l'implant 1 de l'invention, les corps d'ancrage primaire 5 et secondaire 8 sont préférentiellement sensiblement rigides et non déformables (ou uniquement de façon très limitée et localisée), de sorte qu'ils ne se déforment pas de manière significative sous l'action respective des premier 2 et deuxième 3 os. En particulier, les corps d'ancrage primaire 5 et secondaire 8 sont conçus (en particulier en termes de dimensionnement et de choix de matériaux) pour ne pas fléchir respectivement le long des premier A-A' et deuxième B-B' axes, ou pour ne pas se contracter sous l'effet d'une pression axiale ou radiale, lorsqu'ils sont respectivement introduits, et éventuellement ancrés, dans les premier 2 et deuxième 3 os. Ceci étant, le corps d'ancrage secondaire 8 pourra toutefois optionnellement être pourvu d'une lumière 43 ménagée à travers la surface externe secondaire 41 (figure 4).

Débouchant de part et d'autre de ladite surface externe secondaire 41, et de préférence à travers le (les) méplat(s) secondaire(s) 42, cette lumière 43 s'étend de préférence longitudinalement le long du deuxième axe B-B'. Selon les dimensions et positions choisies pour une telle lumière 43, il est alors possible de conférer au corps d'ancrage secondaire 8 une certaine déformabilité (en particulier sous l'effet d'une pression radiale), afin par exemple de faciliter son insertion et son ancrage dans le deuxième os 3. Egalement, une telle lumière 43 pourra avantageusement permettre la formation d'un pont osseux à travers le corps d'ancrage secondaire 8 au cours de l'arthrodèse, favorisant ainsi un ancrage robuste et fiable du corps d'ancrage secondaire 8 dans le deuxième os 3. Bien évidemment, le corps d'ancrage primaire 5 pourra, optionnellement ou alternativement, être pourvu d'une telle lumière.

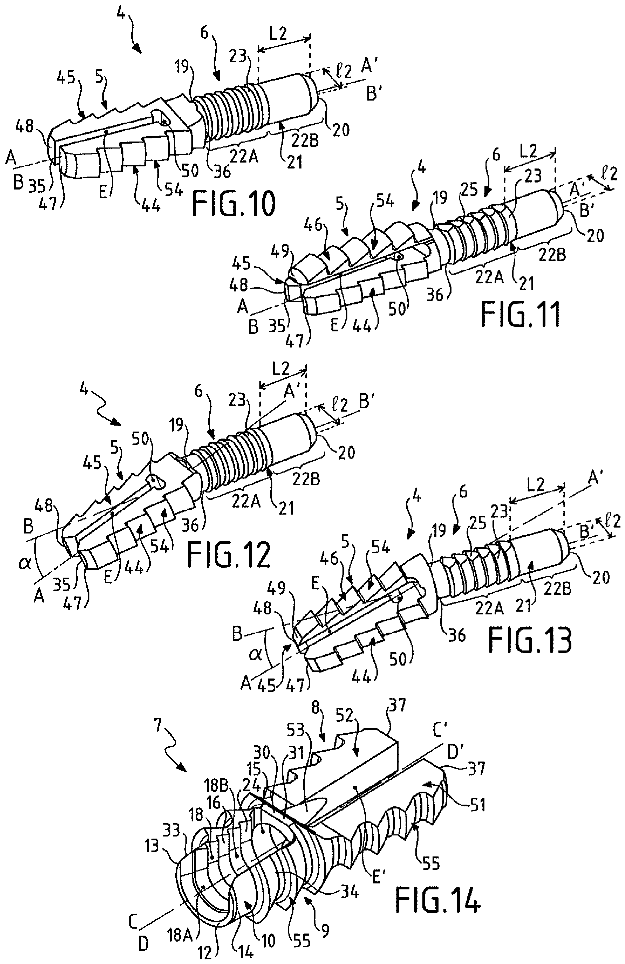

Selon un deuxième mode de réalisation préférentiel de l'implant 1 de l'invention (figures 10 à 17), le corps d'ancrage primaire 5 comprend au moins deux bras d'ancrage primaire 44, 45, 46, qui s'étendent depuis le voisinage de l'extrémité de base primaire 36 en direction de l'extrémité de pénétration primaire 35 du corps d'ancrage primaire 5. De préférence, ces bras d'ancrage primaire 44, 45, 46 sont orientés selon des directions respectives propres, en s'écartant de ladite l'extrémité de base primaire 36. Dans ce cas, le premier axe A-A' correspond alors à la direction d'extension générale moyenne du corps d'ancrage primaire 5, résultant de la combinaison des bras d'ancrage primaires 44, 45, 46. Ces derniers présentent avantageusement chacun une extrémité terminale 47, 48, 49 d'implantation dans le premier os 2. Préférentiellement effilées, coniques, bombées ou chanfreinées pour faciliter l'insertion et la pénétration des bras d'ancrage primaires 44, 45, 46 dans le premier os 2, ces extrémités terminales 47, 48, 49 définissent avantageusement l'extrémité de pénétration primaire 35 du corps d'ancrage primaire 5. Avantageusement, lesdits bras d'ancrage primaires 44, 45, 46 sont conçus pour être flexibles, par exemple de façon élastique. Ainsi, le corps d'ancrage primaire 5 s'adapte, par déformation des bras d'ancrage primaires 44, 45, 46, à la forme du premier os 2, et en particulier de son canal médullaire, afin de pouvoir être ancré de façon fiable, sûre, sans douleur pour le patient. De préférence, les bras d'ancrage primaires 44, 45, 46 sont séparés l'un de l'autre par un espace libre primaire E de manière à pouvoir être rapprochés l'un de l'autre par déformation des bras d'ancrage primaires 44, 45, 46 sous l'action du premier os 2. Une telle conception rend préférentiellement le corps d'ancrage 5 compressible de façon centripète par rapport au premier axe A-A'. L'élasticité des bras d'ancrage primaires 44, 45, 46 permet préférentiellement au corps d'ancrage primaire 5 d'appliquer une pression centrifuge au canal médullaire (ou de façon générale au logement d'ancrage), ce qui favorise la rétention du composant primaire 4 au sein du premier os 2. Le corps d'ancrage primaire 5 est ainsi apte à former une liaison fiable et polyvalente, du composant primaire 4 avec le premier os 2. De préférence, chacun desdits bras d'ancrage primaire 44, 45, 46 présente un côté intérieur formant une face orientée en direction du premier axe A-A' et sensiblement ortho-radiale à ce dernier, un côté extérieur opposé, et deux côtés latéraux reliant le côté extérieur au côté intérieur. De préférence encore, ledit côté extérieur n'est pas ortho-radial au premier axe A-A', mais s'étend au contraire selon une direction sécante à ce dernier, de sorte à définir une forme fictive du corps d'ancrage primaire 5 qui est généralement convergente en direction de l'extrémité de pénétration primaire 35 de ce dernier, le long du premier axe A-A'.

Selon une variante préférentielle (figures 10 et 12), le corps d'ancrage primaire 5 comprend deux bras d'ancrage primaires 44, 45. De préférence, ces bras d'ancrage primaires 44, 45sont disposés symétriquement par rapport à un plan porté par le premier axe A-A'. De préférence, les côtés latéraux de ces bras d'ancrage primaires 44, 45 sont parallèles entre eux, et deux à deux coplanaires, de sorte que la forme desdits bras d'ancrage primaires 44, 45 est en partie délimitée par deux plans parallèles communs auxdits bras d'ancrage primaires 44, 45. Les côtés latéraux définissent ainsi avantageusement des méplats qui contribuent à bloquer le composant primaire 4 en rotation autour du premier axe A-A' par rapport au premier os 2.

Selon une autre variante de l'invention (figures 11 et 13), le corps d'ancrage primaire 5 comprend trois bras d'ancrage primaires 44, 45, 46, de manière à améliorer la stabilité du composant primaire 4 dans le premier os 2. De préférence, deux de ces bras d'ancrage primaires 44, 45, 46 sont avantageusement conformes ceux de la variante préférentielle exposée ci-dessus. Avantageusement, le troisième desdits bras d'ancrage primaires 44, 45, 46 sera quant à lui avantageusement disposé de sorte que sa direction propre soit contenue dans le plan de symétrie des deux autres bras d'ancrage primaires 44, 45, 46. Bien entendu, le corps d'ancrage primaire 5 pourra comporter davantage de bras d'ancrage primaires, et / ou présenter des configurations relatives différentes de ces derniers, sans sortir pour autant du cadre de l'invention. Optionnellement, au moins l'un des bras d'ancrage primaires 44, 45, 46 pourra présenter une zone 50 de section réduite favorisant la déformation dudit bras d'ancrage primaire 44, 45, 46 par flexion de ce dernier au niveau de ladite zone 50 de section réduite, en direction ou à l'écart d'un autre desdits bras d'ancrage primaires 44, 45, 46 (figures 10 à 13). Cette zone 50 de section réduite forme de préférence une encoche ménagée dans le bras primaire 44, 45, 46, au voisinage de l'extrémité de base primaire 36, l'encoche étant avantageusement ouverte sur l'espace libre primaire E, de préférence de façon à ce que le bras d'ancrage primaires 44, 45, 46 concerné puisse être fléchi au niveau de sa zone 50 de section réduite pour converger vers un autre des bras d'ancrage primaires 44, 45, 46.

Selon ce deuxième mode de réalisation préférentiel de l'implant 1 de l'invention (figure 14), le corps d'ancrage secondaire 8 comprend quant à lui avantageusement deux bras d'ancrage secondaires 51, 52, qui s'étendent depuis le voisinage de l'extrémité de base secondaire 38 en direction de l'extrémité de pénétration secondaire 37 du corps d'ancrage secondaire 8, et qui sont préférentiellement séparés l'un de l'autre par un espace libre secondaire E' de manière à pouvoir être rapprochés l'un de l'autre par déformation desdits bras d'ancrage secondaires 51, 52 sous l'action du deuxième os 2. Avantageusement, ces deux bras d'ancrage secondaires 51, 52 sont sensiblement conformes à la description qui a été faite plus haut des deux bras d'ancrage primaires 44, 45 de la variante préférentielle des figures 10 et 12, aux éventuelles différences de dimensionnement près, notamment de leur section et leur longueur. Egalement, l'espace libre secondaire E' pourra être plus étroit (ou plus large) que ledit espace libre primaire E. De préférence, les côtés latéraux de ces deux bras d'ancrage secondaires 51, 52 sont parallèles entre eux, et deux à deux coplanaires, de sorte que la forme des deux bras d'ancrage secondaires 51, 52 est en partie délimitée par deux plans parallèles communs auxdits deux bras d'ancrage secondaires 51, 52. Les côtés latéraux définissent ainsi avantageusement des méplats qui contribuent à bloquer le composant secondaire 5 en rotation autour du troisième axe C-C' par rapport au deuxième os 3. Bien entendu, le corps d'ancrage secondaire 8 pourra comporter davantage de bras d'ancrage secondaires, et / ou présenter des configurations relatives différentes de ces derniers, sans sortir pour autant du cadre de l'invention.

La figure 14 illustre une variante préférentielle particulièrement intéressante de ce deuxième mode de réalisation préférentiel de l'implant 1 de l'invention. Selon cette variante préférentielle, le connecteur femelle 9 et le corps d'ancrage secondaire 8 forment une pièce monobloc d'un seul tenant, et le logement 15 du connecteur femelle 9 débouche de l'extrémité arrière 11 du corps principal 10 du connecteur femelle 9 pour autoriser le passage du connecteur mâle 6. En d'autres termes, la paroi de fond 17 du logement 15 est avantageusement confondue avec ladite extrémité arrière 11 et est pourvue d'un trou traversant (non visible aux figures), qui est configuré, conformé et dimensionné pour autoriser le passage, de préférence par translation selon le quatrième axe D-D', du connecteur mâle 6 et, plus précisément encore, de la deuxième portion 22B de ce dernier. Selon cette variante, le logement 15 ou le connecteur mâle 6 est dimensionné de telle manière qu'une partie au moins de ladite deuxième portion 22B puisse saillir du corps principal 10 par ledit trou traversant, lorsque les composants primaire 4 et secondaire 7 sont reliés ensemble. Pour ce faire, on pourra par exemple prévoir que la longueur du logement 15 selon le quatrième axe D-D' est inférieure à la longueur du connecteur mâle 6 selon le deuxième axe B-B'. Toujours selon cette variante, l'espace libre secondaire E' qui sépare lesdits bras d'ancrage secondaires 51, 52 est avantageusement choisi de dimension inférieure à l'encombrement transversal maximal ℓ2 de la deuxième portion 22B du connecteur mâle 6, tel que celui-ci a été défini ci-avant. En outre, au moins l'un des bras d'ancrage secondaires 51, 52 présente avantageusement une zone d'appui 53 inclinée favorisant la déformation dudit bras d'ancrage secondaire 51, 52 par flexion de ce dernier au niveau de ladite zone d'appui 53 inclinée, à l'écart d'un autre desdits bras d'ancrage 51, 52 secondaires, sous l'action du connecteur mâle 6. Tel qu'illustré, la zone d'appui 53 inclinée forme de préférence une encoche ménagée dans le bras secondaire 51, 52, au voisinage de l'extrémité de base secondaire 38. De préférence, cette encoche présente une forme générale conique ou au moins convergente en direction de l'extrémité de pénétration secondaire 37 du corps d'ancrage secondaire 8. En outre, ladite encoche est avantageusement ouverte sur l'espace libre E', de préférence de façon à ce que le bras d'ancrage secondaire 51, 52 concerné puisse être fléchi au niveau de sa section d'appui 53 de manière à s'écarter de l'autre bras d'ancrage secondaire 51, 52, sous l'action de l'extrémité libre 21 et de la deuxième portion 22B du connecteur mâle 8 envahissant l'espace libre secondaire E'. Il est ainsi l'intermédiaire des connecteurs mâle 6 et femelle 9, de forcer l'écartement relatif des bras secondaires 51, 52 et de renforcer de la sorte l'ancrage du composant secondaire 7 dans ledit deuxième os 2. Inversement, lorsque les moyens de verrouillage autorisent un verrouillage réversible des connecteurs mâle 6 et femelle 9, le retrait du connecteur mâle 8 permettra avantageusement aux bras secondaires 51, 52 de revenir en position l'un vers l'autre et de relâcher tout ou partie de l'effort d'ancrage qu'ils exercent dans le deuxième os 3. Dès lors, le composant secondaire 7 pourra alors être aisément extrait du deuxième os 3.

Bien évidemment, les premier et deuxième modes de réalisation préférentiel exposés ci-avant ne sont pas nécessairement exclusifs l'un de l'autre, et il est parfaitement envisageable que l'implant 1 de l'invention comprenne un composant primaire 4 selon le premier mode et un composant secondaire 6 selon le deuxième mode ou, réciproquement, un composant secondaire 6 selon le deuxième mode et un composant primaire 4 selon le deuxième mode.

Par ailleurs, lesdits corps d'ancrage primaire 5 et secondaire 8 sont préférentiellement respectivement pourvus d'éléments de rétention primaire 54 et secondaire 55 desdits corps d'ancrage primaire 5 et secondaire 8 respectivement dans les premier 2 et deuxième 3 os. Eventuellement déformables sous l'action respective des premier 2 et deuxième 3 os, ces éléments de rétention primaire 54 et secondaire 55 permettent avantageusement d'améliorer la fiabilité et la robustesse de l'ancrage respectif des corps d'ancrage primaire 5 et secondaire 8 dans les premier 2 et deuxième 3 os.

Selon le premier mode de réalisation préférentiel de l'implant 1 de l'invention, l'élément de rétention primaire 54 est formé par au moins une ligne de dents de rétention primaires qui s'étendent le long du corps d'ancrage primaire 5 en faisant saillie de la surface externe primaire 44 de ce dernier, tandis que l'élément de rétention secondaire 55 est formé par une au moins ligne de dents de rétention secondaires qui s'étendent le long du corps d'ancrage secondaire 8 en faisant saillie de la surface externe secondaire 41 de ce dernier. Dans l'exemple des figures 1 à 9, les éléments de rétention primaire 54 et secondaire 55 sont de préférence respectivement formés de deux lignes de dents de rétention primaires, qui s'étendent chacune le long de faces latérales du corps d'ancrage primaire 5 qui relient les faces portants les méplats primaires 40, et de deux lignes de dents de rétention secondaires, qui s'étendent chacune le long de faces latérales du corps d'ancrage secondaire 8 qui relient les faces portants lesdits méplats secondaires 42. De préférence, le connecteur femelle 9 étant prévu pour être inséré intégralement dans le deuxième os 3, il est également avantageusement pourvu d'un élément de rétention secondaire 55, avantageusement formé par une au moins une ligne de dents de rétention secondaires qui s'étendent le long du corps principal 10 en faisant saillie de la paroi latérale 14 de ce dernier. De préférence encore, le connecteur femelle 9 est pourvu de deux lignes de dents de rétention secondaires, chacune s'étendant respectivement axialement le long de la troisième 33 et la quatrième 34 faces latérales du corps principal 10 (figures 4 et 5).

Selon le deuxième mode de réalisation préférentiel de l'implant 1 de l'invention, chacun des bras d'ancrage primaires 44, 45, 46 est avantageusement pourvu d'un élément primaire 54 de rétention dudit bras d'ancrage primaire 44, 45, 46 dans le premier os 2. Respectivement, chacun des bras d'ancrage secondaire 51, 52 est avantageusement pourvu d'un élément secondaire 55 de rétention dudit bras d'ancrage secondaire 51, 52 dans le deuxième os 3. L'élément de rétention primaire 54 est préférentiellement formé par une ligne de dents de rétention primaires qui s'étendent le long du bras d'ancrage primaire 44, 45, 46, et font saillie de ce dernier de façon centrifuge par rapport à l'espace libre primaire E. Respectivement, l'élément de rétention secondaire 55 est préférentiellement formé par une ligne de dents de rétention secondaires qui s'étendent le long du bras d'ancrage secondaire 51, 52, et font saillie de ce dernier de façon centrifuge par rapport à l'espace libre secondaire E'.

Ainsi, l'ancrage des composants primaire 4 et secondaire 6 dans les os du patient est ainsi particulièrement fiable et durable, tout en étant facile à effectuer.

Selon une variante préférentielle du composant primaire 5 (figures 1, 2, 10 et 11), le corps d'ancrage primaire 5 s'étend de préférence longitudinalement selon le premier axe A-A', et le connecteur mâle 8 s'étend longitudinalement dans un sens opposé à celui du corps d'ancrage primaire 5 selon le deuxième axe B-B', lequel est préférentiellement confondu avec le premier axe A-A'. Le corps d'ancrage primaire 6 s'étend donc de manière coaxiale avec le connecteur mâle 8, et le composant primaire 5 forme ainsi avantageusement un composant secondaire 5 sensiblement droit, d'extension moyenne sensiblement rectiligne. Avantageusement, un tel composant primaire 4 pourra être combiné à un composant secondaire 7 sensiblement droit, c'est-à-dire à un composant secondaire 7 dont le corps d'ancrage secondaire 8 et le connecteur femelle 9 s'étendent respectivement longitudinalement selon des troisième C-C' et quatrième D-D' axes sensiblement confondus. On obtient alors avantageusement un implant 1 d'arthrodèse droit, les corps d'ancrage primaire 5 et secondaire 6 et les connecteurs mâle 8 et femelle 9 s'étendant alors longitudinalement de préférence selon une seule et même direction (figure 1). Un tel implant 1 est particulièrement adapté dans le cas où le premier os 2 et le deuxième os 3 doivent être disposés de façon coaxiale en vue de leur fusion.

Selon une autre variante préférentielle du composant primaire 4 (figures 3, 12 et 13), le corps d'ancrage primaire 5 s'étend de préférence longitudinalement selon le premier axe A-A', et le connecteur mâle 8 s'étend longitudinalement dans un sens opposé à celui du corps d'ancrage primaire 5 selon le deuxième axe B-B', lequel est préférentiellement sécant au premier axe A-A'. De préférence, le deuxième axe B-B' est alors avantageusement incliné par rapport au premier axe A-A' selon un angle α d'élévation compris entre 0 et 30° (0 < α ≤ 30°).

Avantageusement, un tel composant primaire 4 pourra être relié à un composant secondaire 6 droit, tel qu'introduit ci-dessus, pour obtenir ainsi un implant 1 d'arthrodèse coudé. Un tel implant 1 est particulièrement adapté dans le cas où le premier os 2 et le deuxième os 3 doivent être disposés selon des directions moyennes sécantes. Bien entendu l'implant 1 pourra être conçu avec une valeur d'angle d'élévation α différente en fonction de la morphologie osseuse et de l'orientation désirée entre le premier os 2 et le deuxième os 3. De préférence, la valeur de l'angle α correspond à une valeur anatomique de l'orientation naturelle (au repos) du premier os 2 par rapport au deuxième os 3.

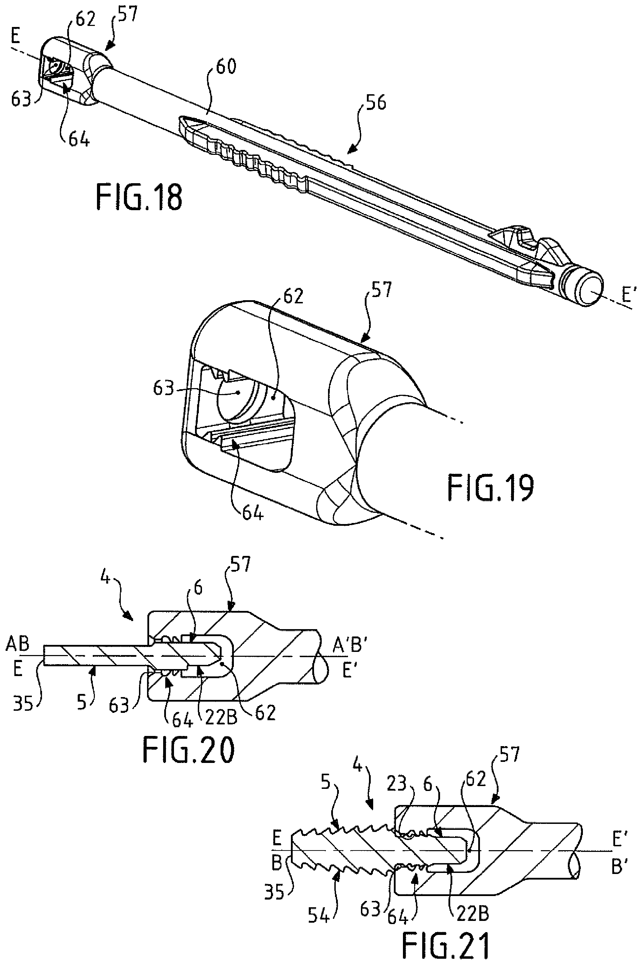

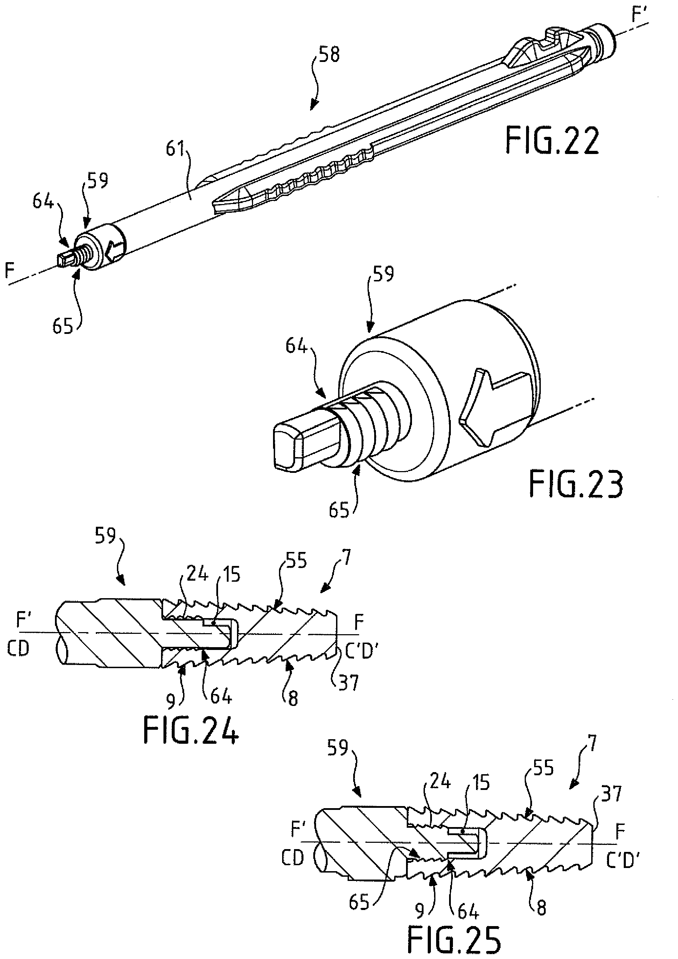

L'invention concerne également, en tant que tel, un kit chirurgical (non illustré) comprenant un implant 1 d'arthrodèse pour favoriser la fusion osseuse d'un premier os 2 avec un deuxième os 3, lequel implant 1 d'arthrodèse est conforme à la description détaillée qui précède. Bien entendu, un tel kit pourra comprendre plus d'un implant 1 conforme à l'invention. Par exemple, pour offrir un large choix opératoire au chirurgien, le kit pourra avantageusement comprendre une pluralité d'implants 1, de tailles et configurations différentes (implant 1 droit, implant 1 coudé, implants à corps d'ancrages pleins ou à bras d'ancrage, etc.). Le kit de l'invention comprend également au moins :
- un premier instrument 56 de préhension pourvu d'un premier embout 57 de solidarisation du composant primaire 4 de l'implant 1 avec le premier instrument 56 de préhension, par l'intermédiaire du connecteur mâle 6 du composant primaire 4 (figures 18 à 21) ;
- un deuxième instrument 58 de préhension pourvu d'un deuxième embout 59 de solidarisation du composant secondaire 7 de l'implant 1 avec le deuxième instrument 58 de préhension, par l'intermédiaire du connecteur femelle 9 du composant secondaire 7 (figures 22 à 25).

Les premier 56 et deuxième 58 instruments de préhension comprennent de préférence respectivement un premier corps principal 60 et un deuxième corps principal 61, par exemple de forme générale allongée rectiligne selon des cinquième E-E' et sixième F-F' axes d'extension longitudinal respectifs, qui se terminent respectivement par les premier 57 et deuxième 59 embouts de solidarisation. De préférence, les premier 60 et deuxième 61 corps principaux sont respectivement conçus pour coopérer un premier et un deuxième manche (ou poignée) amovible (non illustrée) connectable de façon amovible aux premier 60 et deuxième 61 corps principaux.

Les premier 57 et deuxième 59 embouts sont de préférence respectivement conçus pour accueillir le connecteur mâle 6 et le connecteur femelle 9 de façon à ce que le deuxième axe B-B' d'extension du connecteur mâle 6 et, respectivement le quatrième axe D-D' d'extension du connecteur femelle 9, soient confondus avec (ou au moins parallèles à) le cinquième D-D' et le sixième F-F' axe d'extension respectifs desdits premier 60 et deuxième 61 corps principaux. Tel qu'illustré aux figures 18 à 21, le premier embout 57 comprend un évidement 62 interne axial de conformation et configuration complémentaires de celles du connecteur mâle 6 du composant primaire 4. Cet évidement 62 débouche axialement du premier embout 57 par un orifice 63 de formes et dimensions conjuguées à la section du connecteur mâle 6, de sorte que ce dernier peut être introduit (de préférence intégralement) dans l'évidement 62 par l'intermédiaire dudit orifice 63. Tel qu'illustré aux figures 22 à 25, le deuxième embout 59 comprend quant à lui un élément de connexion mâle 64 allongé, de direction d'extension longitudinale moyenne confondue (ou au moins parallèle) au sixième axe F-F' d'extension du deuxième corps principal 61 du deuxième instrument 58 de préhension, et de conformation et configuration complémentaires de celles du connecteur femelle 9 du composant secondaire 7. De préférence, lesdits connecteurs mâle 6 et femelle 9 de l'implant 1 comprenant des moyens de verrouillage complémentaires conçus et configurés pour assurer un verrouillage relatif réversible des composants primaire 4 et secondaire 7 de l'implant 1 par rotation d'un quart de tour du connecteur mâle 6 relativement au connecteur femelle 9 (comme envisagé ci-avant),
- le premier embout 57 de solidarisation est préférentiellement pourvu d'un premier moyen de couplage 64 (par exemple de type dent) coopérant avec le connecteur mâle 6 du composant primaire 4 de l'implant 1 pour solidariser le composant primaire 4 avec le premier instrument 56 de préhension par rotation d'un quart de tour du connecteur mâle 6 du composant primaire 4 relativement au premier embout de solidarisation 57, et respectivement
- le deuxième embout 59 de solidarisation est pourvu d'un deuxième moyen de couplage 65 (par exemple de type cran) coopérant avec le connecteur femelle 9 du composant secondaire 7 de l'implant 1 pour solidariser le composant secondaire 7 avec le deuxième instrument 58 de préhension par rotation d'un quart de tour du composant secondaire 7 relativement au deuxième embout de solidarisation 59.

A ce titre, les premier 57 et deuxième 59 embouts pourront, de manière avantageuse, reprendre respectivement toute ou partie des caractéristiques exposées précédemment des connecteurs mâle 6 et femelle 9, en relation avec un tel verrouillage réversible par rotation d'un quart de tour. En particulier, les premier 64 et deuxième 65 moyens de couplage seront de préférence conçus et configurés pour coopérer respectivement avec le premier moyen de verrouillage du connecteur mâle 6 et avec le deuxième moyen de verrouillage du connecteur femelle 9. De préférence encore, ledit deuxième moyen de couplage 65 est plus spécifiquement conçu et configuré pour ne permettre une rotation d'un quart de tour du connecteur femelle 9 relativement au deuxième embout 59 que selon un seul sens de rotation (par exemple dans le sens horaire) du connecteur femelle 9 autour de son quatrième axe D-D' d'extension. La solidarisation puis la désolidarisation du deuxième 59 embout et du connecteur femelle 9 du composant secondaire 7 de l'implant 1 s'opéreront ainsi avantageusement par une succession de rotations d'un quart de tour dans un même sens de rotation. La rotation en sens inverse sera quant à elle avantageusement bloquée, par exemple par une conformation spécifique du deuxième moyen de couplage 65. Ainsi, les premier 56 et deuxième 58 instruments permettent une préhension et une pose particulièrement aisée, rapide et précise des composants primaire 4 et secondaire 7 respectivement dans le premier os 2 et dans le deuxième os 3.

De préférence, lesdits premiers 56 et deuxième 58 instruments de préhension sont en matériau plastique, tel qu'en polyétheréthercétone PEEK, ce dernier étant optionnellement chargé. Avantageusement, le premier instrument 56 de préhension et le premier embout 57 d'une part, et les deuxième instrument 58 de préhension et le deuxième embout 59 d'autre part, forment une pièce monobloc d'un seul tenant, par exemple obtenue par moulage ou usinage. Alternativement, lesdits premier 57 et deuxième 59 embouts pourront constituer des pièces rapportées, de manière amovible ou non, respectivement auxdits premier 60 et deuxième 61 corps principaux. Bien évidemment, le kit de l'invention pourra avantageusement comprendre d'autres outils ou instruments éventuellement nécessaires à la mise en oeuvre d'au moins un implant 1 conforme à l'invention, tel que par exemple un instrument d'ouverture préférentiellement pourvu d'un embout de perçage des premier 2 et deuxième 3 os.

Enfin, un exemple de procédé chirurgical d'implantation, dans le corps d'un patient, de l'implant 1 décrit précédemment va être présenté ci-après. De manière préférentielle, le procédé comporte notamment tout ou partie des étapes suivantes. On effectue tout d'abord une incision dans le corps d'un patient afin d'atteindre le premier os 2 et le deuxième os 3. On sectionne ensuite l'articulation reliant le premier os 2 au deuxième os 3 afin de dégager les parties extrémales de ces derniers. Après avoir, si nécessaire, procédé à une ablation des éléments cartilagineux présents au niveau de l'articulation, on retire ou perce l'extrémité des premier os 2 et deuxième os 3, afin de mettre à jour leurs canaux médullaires respectifs. On évide ensuite l'intérieur des canaux médullaires de façon partielle ou totale afin de préparer un espace creux libre pour permettre l'introduction des corps d'ancrage primaire 5 et secondaire 7 (ainsi que de préférence, du connecteur femelle 9) de l'implant 1 dans chacun des canaux médullaires. Puis, on procède à une résection osseuse de la zone dorsale de la tête ou extrémité du deuxième os 3, de sorte à ménager une ouverture dorsale de dimensions et forme conjuguées à la deuxième zone 18B de l'ouverture 18 du connecteur femelle 9.

On saisit alors le composant secondaire 6 de l'implant 1 à l'aide du deuxième instrument 58 de préhension, en verrouillant préférentiellement le composant secondaire 6 relativement au deuxième embout 59 de préhension du deuxième instrument 58 par rotation d'un quart de tour dans le sens horaire. Puis, on insère et ancre, optionnellement en force et de préférence intégralement, le composant secondaire 6 dans le canal médullaire du deuxième os 3 (figure 15), de sorte que l'ouverture 18 du connecteur femelle 9 soit positionnée en regard de ladite ouverture dorsale. Ensuite, on désolidarise de préférence le composant secondaire 6 du deuxième instrument 58 par rotation d'un quart de tour dans le sens horaire.

On saisit le composant primaire 4 de l'implant 1 à l'aide du premier instrument 56 de préhension, en verrouillant préférentiellement le composant primaire 4 relativement au premier embout 57 de préhension du premier instrument 56 par rotation d'un quart de tour. Puis, on insère et ancre, optionnellement en force, le composant primaire 4 dans le canal médullaire du premier os 2 (figure 15), par l'intermédiaire du corps d'ancrage primaire 5, de sorte que seul le connecteur mâle 6 fasse saillie hors du premier os 2. Ensuite, on désolidarise de préférence le composant primaire 4 du premier instrument 56 par rotation d'un quart de tour.

On introduit ensuite la deuxième portion 22B (extrémale) du connecteur mâle 6 dans le logement 15 du connecteur femelle 9 de manière latérale, par l'intermédiaire à la fois de la première 18A et la deuxième 18B zones de l'ouverture 18 de ce dernier (figure 16). Puis, on poursuit l'introduction du connecteur mâle 6 dans le logement 15 de sorte à amener ces derniers dans une position relative coaxiale. On exerce ensuite un effort de rapprochement des premier 2 et deuxième 3 os pour permettre une translation axiale (de préférence selon une direction confondue avec les deuxième B-B' et quatrième D-D' axes) du connecteur mâle 6 dans le logement 15 jusqu'à obtenir la coopération et la mise en prise des premier et deuxième moyens de verrouillage relatif des composants primaire 4 et secondaire 7. Avantageusement, on poursuit cet effort de rapprochement des premier 2 et deuxième 3 os pour ajuster progressivement l'écartement relatif des corps d'ancrage primaire 4 et secondaire 7, de préférence encore jusqu'à venir abouter les premier 2 et deuxième 3 os. Dans ce cas, l'extrémité de base primaire 36 du corps d'ancrage primaire 5 vient alors de préférence au contact (ou au voisinage immédiat) de la face avant 12 du corps principal 10 du connecteur femelle 9. Optionnellement, une partie au moins de la deuxième portion 22B dudit connecteur mâle 6 fait alors saillie de l'extrémité arrière 11 dudit corps principal 10 et s'étend entre les bras d'ancrage secondaire 51, 52, forçant la déformation de ces derniers en direction opposée l'un de l'autre (figure 17).

Ce procédé opératoire pourra également éventuellement comporter une étape d'introduction d'un matériau ostéo-inducteur dans les canaux médullaires respectifs des premier 2 et deuxième 3 os, avant insertion au sein de ses derniers des corps d'ancrage primaire 5 et secondaire 7 des composants primaire 4 et secondaire 6. Bien entendu, certaines étapes du procédé décrites ci-avant pourront avantageusement être omises ou effectuées dans un ordre différent.

## Revendications

1. Implant (1) d'arthrodèse pour favoriser la fusion osseuse d'un premier os (2) avec un deuxième os (3), ledit implant (1) comprenant un composant primaire (4) comportant un corps d'ancrage primaire (5) et un connecteur mâle (6), et un composant secondaire (7) comportant un corps d'ancrage secondaire (8) et un connecteur femelle (9), ledit connecteur femelle (9) comprenant :
- un corps principal (10) qui s'étend longitudinalement entre une extrémité arrière (11) reliée audit corps d'ancrage secondaire (8) et une face avant (12) opposée, et qui présente une paroi latérale (14) reliant ladite extrémité arrière (11) à ladite face avant (12) ;
- un logement (15) ménagé au sein dudit corps principal (10) et configuré pour recevoir ledit connecteur mâle (6) afin de relier ensemble lesdits composants primaire (4) et secondaire (7),
ledit connecteur femelle (9) comprenant en outre une ouverture (18) qui est ménagée dans ledit corps principal (10), en communication avec ledit logement (15), et qui s'étend de manière continue à la fois sur ladite face avant (12) et ladite paroi latérale (14), et qui est dimensionnée et configurée pour autoriser une introduction latérale du connecteur mâle (6) dans ledit logement (15) par ladite ouverture (18),
lesdits composant primaire (4) et secondaire (6) formant chacun une pièce monobloc d'un seul tenant, et lesdits corps d'ancrage primaire (5) et secondaire (8) étant respectivement conçus pour assurer une insertion et un ancrage du composant primaire (4) dans le premier os (2), et du composant secondaire (7) dans le deuxième os (3), par impaction ou par coincement en force.

2. Implant (1) selon la revendication précédente, **caractérisé en ce que** les connecteurs mâle (6) et femelle (9) comprennent respectivement un premier et un deuxième moyens de verrouillage complémentaires qui coopèrent pour assurer le verrouillage relatif des composants primaire (4) et secondaire (7).

3. Implant (1) selon la revendication précédente, **caractérisé en ce que** lesdits premier et deuxième moyens de verrouillage complémentaires sont conçus et configurés pour assurer un verrouillage relatif réversible des composants primaire (4) et secondaire (7).

4. Implant (1) selon la revendication 2 ou 3, **caractérisé en ce que** lesdits premier et deuxième moyens de verrouillage complémentaires forment un système de dents (23) et de crans (24) étagés complémentaires autorisant un ajustement progressif de l'écartement relatif des corps d'ancrage primaire (5) et secondaire (7).

5. Implant (1) selon l'une quelconque des revendications précédentes, **caractérisé en ce que** le connecteur mâle (6) s'étend longitudinalement entre une première extrémité (19) reliée au corps d'ancrage primaire (5) et une deuxième extrémité (20) libre, et **en ce que** le connecteur mâle (6) comprend une première portion (22A) et une deuxième portion (22B), la première portion (22A) s'étendant depuis ladite première extrémité (19), tandis que la deuxième portion (22B) prolonge ladite première portion (22A) jusqu'à ladite deuxième extrémité (20) libre.

6. Implant (1) selon la revendication précédente, **caractérisé en ce que** ladite ouverture (18) du connecteur femelle (9) comprend une première zone (18A) qui s'étend sur ladite face avant (12) et une deuxième zone (18B) qui s'étend sur ladite paroi latérale (14) du corps principal (10) du connecteur femelle (9), ladite deuxième zone (18B) d'ouverture étant conformée et configurée pour autoriser une introduction latérale de ladite deuxième portion (22B) du connecteur mâle (6) dans ledit logement (15) par ladite deuxième zone (18B) de l'ouverture (18).

7. Implant (1) selon la revendication précédente et l'une quelconque des revendications 2 à 4, **caractérisé en ce que** ledit premier moyen de verrouillage est porté par la première portion (22A) du connecteur mâle (6) uniquement.

8. Implant (1) selon l'une quelconque des revendications précédentes, **caractérisé en ce que** le corps d'ancrage primaire (5) s'étend longitudinalement entre une extrémité de pénétration primaire (35) dans le premier os (2) et une extrémité de base primaire (36) opposée, et présente une surface externe primaire (44) reliant l'extrémité de base primaire (41) à l'extrémité de pénétration primaire (40), la surface externe primaire (44) étant de forme généralement convergente, par exemple conique, en direction de l'extrémité de pénétration primaire (35).

9. Implant (1) selon la revendication précédente, **caractérisé en ce que** ledit corps d'ancrage primaire (5) est pourvu d'au moins un méplat primaire (40) longitudinal ménagé dans ladite surface externe primaire (41).

10. Implant (1) selon l'une quelconque des revendications précédentes, **caractérisé en ce que** le corps d'ancrage secondaire (8) s'étend longitudinalement entre une extrémité de pénétration secondaire (37) dans le deuxième os (3) et une extrémité de base secondaire (38) opposée, et présente une surface externe secondaire (41) reliant l'extrémité de base secondaire (38) à l'extrémité de pénétration secondaire (37), la surface externe secondaire (47) étant de forme généralement convergente, par exemple conique, en direction de l'extrémité de pénétration secondaire (37).

11. Implant (1) selon la revendication précédente, **caractérisé en ce que** ledit corps d'ancrage secondaire (8) est pourvu d'au moins un méplat secondaire (42) longitudinal ménagé dans ladite surface externe secondaire (41).

12. Implant (1) selon l'une quelconque des revendications précédentes, **caractérisé en ce que** le corps d'ancrage primaire (5) s'étend longitudinalement selon un premier axe (A-A'), le connecteur mâle (6) s'étendant longitudinalement dans un sens opposé à celui dudit corps d'ancrage primaire (5) selon un deuxième axe (B-B') qui est confondu avec ledit premier axe (A-A').

13. Implant (1) selon l'une quelconque des revendications 1 à 12, **caractérisé en ce que** le corps d'ancrage primaire (5) s'étend longitudinalement selon un premier axe (A-A'), le connecteur mâle (6) s'étendant longitudinalement dans un sens opposé à celui dudit corps d'ancrage primaire (5) selon un deuxième axe (B-B') qui est sécant audit premier axe (A-A'), le deuxième axe (B-B') étant incliné par rapport audit premier axe (A-A') selon un angle (α) d'élévation compris entre 0 et 30°.

14. Kit chirurgical comprenant un implant (1) d'arthrodèse pour favoriser la fusion osseuse d'un premier os (2) avec un deuxième os (3), ledit implant (1) d'arthrodèse étant conforme à l'une quelconque des revendications 1 à 13, et ledit kit comprenant également :
- un premier instrument (56) de préhension pourvu d'un premier embout (57) de solidarisation du composant primaire (4) de l'implant (1) avec le premier instrument (56) de préhension, par l'intermédiaire du connecteur mâle (6) du composant primaire (4) ;
- un deuxième instrument (58) de préhension pourvu d'un deuxième embout (59) de solidarisation du composant secondaire (7) de l'implant (1) avec le deuxième instrument (58) de préhension, par l'intermédiaire du connecteur femelle (9) du composant secondaire (7).

15. Kit selon la revendication précédente, **caractérisé en ce que** :
- le premier embout (57) de solidarisation est pourvu d'un premier moyen de couplage (64) coopérant avec le connecteur mâle (6) du composant primaire (4) de l'implant (1) pour solidariser le composant primaire (4) avec le premier instrument (56) de préhension par rotation d'un quart de tour du connecteur mâle (6) relativement au premier embout (57) de solidarisation, et
- le deuxième embout (59) de solidarisation est pourvu d'un deuxième moyen de couplage (65) coopérant avec le connecteur femelle (9) du composant secondaire (7) de l'implant (1) pour solidariser le composant secondaire (7) avec le deuxième instrument (58) de préhension par rotation d'un quart de tour du composant secondaire (7) relativement au deuxième embout (59) de solidarisation.
